(19)

Europäisches
Patentamt
European
Patent Office
Office européen
des brevets

(11) **EP 1 708 565 B1**

(12) **EUROPEAN PATENT SPECIFICATION**

(45) Date of publication and mention
of the grant of the patent:
**06.06.2012 Bulletin 2012/23**

(21) Application number: **04770459.8**

(22) Date of filing: **31.08.2004**

(51) Int Cl.:
*C07K 14/705* (2006.01)        *G01N 33/50* (2006.01)
*A01K 67/00* (2006.01)

(86) International application number:
**PCT/IL2004/000788**

(87) International publication number:
**WO 2005/025310 (24.03.2005 Gazette 2005/12)**

(54) **MODEL SYSTEM FOR TCR XI CHAIN DOWNREGULATION**

MODELLSYSTEM ZUR ANALYSE DER VERMINDERTEN EXPRESSION DER TCR XI-KETTEN

SYSTEME DE MODELE POUR SOUS-REGULATION DE CHAINE TCR XI

(84) Designated Contracting States:
**AT BE BG CH CY CZ DE DK EE ES FI FR GB GR HU IE IT LI LU MC NL PL PT RO SE SI SK TR**

(30) Priority: **18.09.2003 IL 15801103**

(43) Date of publication of application:
**11.10.2006 Bulletin 2006/41**

(73) Proprietor: **YISSUM RESEARCH DEVELOPMENT COMPANY OF THE HEBREW UNIVERSITY OF JERUSALEM**
**91390 Jerusalem (IL)**

(72) Inventor: **Baniyash, Michal**
**French Hill,**
**97892 Jerusalem (IL)**

(74) Representative: **Howard, Paul Nicholas et al**
**Carpmaels & Ransford**
**One Southampton Row**
**London**
**WC1B 5HA (GB)**

(56) References cited:
• **GARCIA C S ET AL: "Downregulation of the T cell receptor zeta chain in chronically stimulated Jurkat T cells" PROCEEDINGS OF THE AMERICAN ASSOCIATION FOR CANCER RESEARCH ANNUAL MEETING, vol. 39, March 1998 (1998-03), page 85, XP001204799 & 89TH ANNUAL MEETING OF THE AMERICAN ASSOCIATION FOR CANCER RESEARCH; NEW ORLEANS, LOUISIANA, USA; MARCH 28-APRIL 1, 1998 ISSN: 0197-016X**

• **HOURI-HADDAD Y ET AL: "Repeat bacterial challenge in a subcutaneous chamber model results in augmented tumour necrosis factor-alpha and interferon-gamma response, and suppression of interleukin-10" IMMUNOLOGY, vol. 99, no. 2, February 2000 (2000-02), pages 215-220, XP002319325 ISSN: 0019-2805**

• **BRONSTEIN-SITTON NOEMI ET AL: "Expression of the T cell antigen receptor zeta chain following activation is controlled at distinct checkpoints. Implications for cell surface receptor down-modulation and re-expression" JOURNAL OF BIOLOGICAL CHEMISTRY, vol. 274, no. 33, 13 August 1999 (1999-08-13), pages 23659-23665, XP002319326 ISSN: 0021-9258**

• **RODRIGUEZ PAULO C ET AL: "L-arginine consumption by macrophages modulates the expression of CD3zeta chain in T lymphocytes." JOURNAL OF IMMUNOLOGY, vol. 171, no. 3, 1 August 2003 (2003-08-01), pages 1232-1239, XP002319328 ISSN: 0022-1767**

• **VALITUTTI SALVATORE ET AL: "Degradation of T cell receptor (TCR)-CD3-zeta complexes after antigenic stimulation" JOURNAL OF EXPERIMENTAL MEDICINE, vol. 185, no. 10, 1997, pages 1859-1864, XP008043318 ISSN: 0022-1007**

• **LIU HAIYAN ET AL: "On the dynamics of TCR: CD3 complex cell surface expression and downmodulation" IMMUNITY, vol. 13, no. 5, November 2000 (2000-11), pages 665-675, XP002319260 ISSN: 1074-7613**

- BERG LOUISE ET AL: "Granulocytes downregulate T-cell receptor zeta chain by cell contact dependent mechanisms" IMMUNOLOGY LETTERS, vol. 73, no. 2-3, September 2000 (2000-09), page 153, XP008043329 & 24TH EUROPEAN IMMUNOLOGY MEETING OF THE EUROPEAN FEDERATION OF IMMUNOLOGICAL SOCIETIES (EFIS); POZNAN, POLAND; SEPTEMBER 23-26, 2000 ISSN: 0165-2478
- BRONSTEIN-SITTON NOEMI ET AL: "Sustained exposure to bacterial antigen induces interferon-gamma-dependent T cell receptor zeta down-regulation and impaired T cell function." NATURE IMMUNOLOGY, vol. 4, no. 10, October 2003 (2003-10), pages 957-964, XP008043176 ISSN: 1529-2908
- BANIYASH MICHAL: "Tcr zeta-chain downregulation: Curtailing an excessive inflammatory immune response" NATURE REVIEWS IMMUNOLOGY, vol. 4, no. 9, September 2004 (2004-09), pages 675-687, XP008043182 ISSN: 1474-1733
- P. Isomäki ET AL: "Prolonged exposure of T cells to TNF down-regulates TCR zeta and expression of the TCR/CD3 complex at the cell surface.", Journal of immunology, vol. 166, no. 9, 1 May 2001 (2001-05-01), pages 5495-5507, XP55002397,
- COPE A P: "Studies of T-cell activation in chronic inflammation", ARTHRITIS RESEARCH, CURRENT SCIENCE, LONDON, GB, vol. 4, no. Suppl. 3, 9 May 2002 (2002-05-09), pages S197-S211, XP008114976, ISSN: 1465-9905, DOI: 10.1186/AR557

Remarks:

    The file contains technical information submitted after the application was filed and not included in this specification

**Description**

**Field of the Invention**

The present invention relates generally to the field of immunology and clinical immunology, and more specifically to T cell antigen receptors (TCRs). In particular, the present disclosure is concerned with providing a model system characterized by the downregulation of TCR ζ expression and impaired T cell function, which can be very useful for the screening of drugs inducing and/or preventing such an effect. In addition, the invention provides the use of TCR ζ, expression as a marker or as a prognostic marker for immunosuppressive environments, as defined in the claims.

**Background of the Invention**

The TCR is a multi-subunit complex composed of the α/β heterodimer, which is involved in antigen recognition, and the invariant CD3 (γ, δ, ε) chains and ζ-ζ homodimer, that couple antigen recognition to intracellular signaling pathways [Weiss, A. & Littman, D. R., (1994) Cell 76, 263-74, Klausner, R. D. et al. (1990) Annu Rev Cell Biol 6, 403-31]. The TCR ζ chain is considered the limiting factor in TCR assembly and expression, in addition to being crucial to the receptor signaling [Mizoguchi, H. et al. (1992) Science 258, 1795-8; Kane, L. P. et al. (2000) Curr Opin Immunol 12, 242-9]. T cells isolated from hosts (mice and human) bearing various tumors such as renal [Alberola-Ila, J. et al. (1997), Annu Rev Immunol 15, 125-54; Finke, J. H. et al. (1993) Cancer Res 53, 5613-6], colorectal [Nakagomi, H. et al. (1993) Cancer Res 53, 5610-2 ; Matsuda, M. et al. (1995) Int J Cancer 61, 765-72], ovarian [Lai, P. et al. (1996) Clin Cancer Res 2, 161-73], cervical [Kono, K. et al. (1996) Clin Cancer Res 2, 1825-8], breast [Kurt, R. A. et al.(1998) Int J Cancer 78, 16-20], head & neck [Kuss, I. et al. (1999) Clin Cancer Res 5, 329-34] and prostate [Healy, C. G. et al. (1998) Cytometry 32, 109-19] are ζ-deficient and immunologically nonfunctional. In recent years, a similar phenomenon was described in several infectious diseases, such as HIV [Trimble, L. A. & Lieberman, J. (1998) Blood 91, 585-94] and leprosy [Zea, A. H. et al. (1998) Infect Immun 66, 999-504], and also in autoimmune disorders, for example rheumatoid arthritis [Maurice, M. M. et al. (1997) J Immunol 159, 2973-8] and systemic lupus erythematosus [Liossis, S. N. et al. (1998) J Clin Invest 101, 1448-57]. In all these cases, the ζ chain was the sole TCR component that was affected. Thus, although the various pathologies differ in their etiology and physiology, they all show downregulation of TCR ζ expression and impaired *in vitro* T cell function. However, none of these studies delineated the *in vivo* immunological mechanism underlying this phenomenon.

The inventors hypothesized that common factors inherent to all of these pathologies could account for the observed phenomenon, rather than a specific tumor, pathogen or auto-antigen present in each disease. Possibly, sustained exposure to antigen and chronic inflammation may be responsible for the induction of TCR ζ chain downregulation and impaired *in vitro* T cell function, resulting in the attenuation of a chronically activated immune response. To test this premise, the inventors established an *in vivo* experimental system in which healthy mice were repeatedly exposed to the bacterial antigen *Porphyromonas gingivalis* (*P. gingivalis*), which induces a TH1-dominant immune response, resulting in local and systemic inflammation [Genco, C. A. & Arko, R. J. (1994) Methods Enzymol 235, 120-40; Houri-Haddad, Y. et al. (2000) Immunology 99, 215-20].

Cope et al. (2002; Arthritis Res 4 (suppl 3), 197-211) discusses that the exposure of T cell hybridoma cells to TNF results in impaired assembly and stability of the TCR/CD3 complex at the cell surface.

Isomäki et al. (2001, J. Immunol. 166, 5495-5507) teaches that chronic exposure of T cell hybridoma cells to TNF results in a reversible loss of TCRζ chain expression and impaired TCR/CD3 assembly.

In view of the results herein described, it is an object of the present disclosure to provide an *in vivo* model system that mimics the above-mentioned pathologies, wherein TCR ζ is downregulated and T cell function is impaired.

The inventors demonstrated that in this animal model, chronically exposing mice to antigens caused the downregulation of TCR ζ, accompanied by impaired T cell function. Said downregulation is IFNγ-dependent, which makes this a good model system for the study, amongst others, of IFNγ function in the immune system.

**Summary of the Invention**

In the present study, the inventors describe an in vivo model or experimental system, which mimics a situation of chronic systemic inflammation, wherein the exposure of animals to antigens induces the appearance of an immunosuppressive environment, leading to T cell antigen receptor (TCR) ζ chain downregulation and impaired T cell function.

Thus, in a first aspect, the disclosure refers to an in vivo model system for TCR ζ downregulation, wherein said model system is an animal model, preferably mouse, and wherein said animal has been injected with an antigen, said antigen being preferably in admixture with an adjuvant.

In a second aspect, in the model system, the TCR ζ downregulation is associated with the following properties: (a) it is IFNγ-dependent; (b) it requires sustained exposure to the antigen; (c) it requires the development of a TH1-dependent inflammatory immune response; and (d) it correlated with impaired T cell function mediated via the TCR.

In a third aspect, the model system is valuable as a platform for screening of substances that can upregulate or prevent the downregulation of the TCR ζ chain, either by directly affecting the T cells or by neutralizing the inhibitory, i.e. immunosuppressive, environment.

In another aspect, the model system may be applied for assessing the feasibility of immunotherapies, i.e., for evaluating the sensitivity of cells to be used in immunotherapy to the environment of the model system.

Similarly, the model system may be applied for assessing the efficiency of various vaccination protocols when given to a subject carrying an immunosuppressive environment.

The present disclosure also provides a model system for the study of IFNγ function in the immune response.

The model system herein described mimics the immunosuppression caused by cancer, autoimmune disorders and infectious diseases.

In a further aspect, the present disclosure provides a method for generating the model system herein described, comprising the following steps:

(a) exposing healthy animals to an antigen, said antigen being preferably in admixture with an adjuvant;

(b) inducing chronic systemic inflammation; and

(c) evaluating the expression of TCR chains in the T lymphocytes of the antigen-exposed animals;

(d) evaluating T lymphocyte function (*in vivo* or *ex vivo*) in the antigen-exposed animals;

wherein the expression of TCR ζ is downregulated and the expression of TCR α, β, CD3 γ, δ and ε is unchanged compared to a non-exposed animal.

In an even further aspect, the present disclosure refers to a method of screening for substances that can upregulate or prevent the downregulation of the TCR ζ chain *in vivo*, comprising the following steps:

(a) exposing the *in vivo* model system of the disclosure to a test substance;

(b) evaluating the expression of TCR chains in the T lymphocytes of the test substance-exposed animal;

wherein, if the expression of TCR ζ is higher than the expression of TCR ζ in a model system which has not been exposed to said test substance, said test substance is an inhibitor of the downregulation of TCR ζ.

Similarly, the disclosure also enables two *ex vivo* screening methods for substances that can upregulate or prevent the downregulation of the TCR ζ chain in *vitro*.

The first *ex vivo* screening method comprises the following steps:

(a) obtaining a body fluid and/or tissue sample from an animal model system for TCR ζ downregulation, as defined in the disclosure;

(b) separating non-T cells from said sample;

(c) providing T cells from a healthy animal;

(d) establishing a mixed cell culture with said non-T and T cells, wherein, after incubating these two cell populations together, TCR ζ expression is downregulated in said T cells;

(e) exposing said cell culture to a test substance for an effective time period;

(f) evaluating the expression of TCR chains in the T cells in the culture;

wherein if the expression of TCR ζ in said T cells is higher than in the T cells of a non-exposed corresponding mixed culture, said test substance is an inhibitor of the downregulation of TCR ζ expression.

In one preferred embodiment of this first screening method, said body fluid and/or tissue sample is spleen or peripheral blood.

In the second *ex vivo* screening method, both T and non-T cells are derived from a model system animal (i.e., both cell populations were exposed to the antigen *in vivo*). Accordingly, said second *ex* vivo screening method comprises the following steps:

(a) obtaining a body fluid and/or tissue sample from an animal model system for TCR ζ downregulation, as defined in the disclosure;

(b) separating T and non-T cells from said sample;

(c) establishing a mixed cell culture with said non-T and T cells, wherein, after incubating these two cell populations together, TCR ζ expression is downregulated in said T cells;

(d) exposing said cell culture to a test substance for an effective time period;

(e) evaluating the expression of TCR chains in the T lymphocytes in the culture;

wherein if the expression of TCR ζ in said T cells is higher than in the T cells of a non-exposed corresponding mixed culture, said test substance is an inhibitor of the downregulation of TCR ζ expression.

Most preferably, the T cells are obtained from spleen and/or peripheral blood. In addition, it is important to mention that the non-T cells employed in the both screening methods described herein are myeloid cells.

Hence, the present disclosure provides the use of the model system of the invention for screening of substances that can upregulate or prevent the downregulation of the TCR ζ chain.

The present disclosure provides the use of the model system described herein for screening of cells, as well as vaccines, to be used in immunotherapeutic regimens, Such a screening shall be very useful in (a) testing the sensitivity of the cells to the immunosuppressive environment, (b) determining the mode of vaccination, and (c) determining the timing and the mode of the applied immunotherapy, by measuring TCR ζ chain expression.

The present invention also provides the use of TCR ζ chain protein level as a marker for an immunosuppressive environment in a subject suffering from a chronic inflammatory condition, wherein the detection of downregulation of TCR ζ chain protein level in a sample from said subject means the presence of an immunosuppressive environment in said subject. Preferably, said immunosuppressive environment is a result of any one of the following conditions: chronic inflammation, cancer, infections and autoimmune disorders.

In addition, the present invention provides the use of TCR ζ chain protein level as a prognostic marker for the emergence of an immunosuppressive environment in a subject suffering from a chronic inflammatory condition, wherein the detection of dowregulation of TCR ζ chain protein level in a sample from said subject means the development of an immunosuppressive environment.

In a last aspect, the present disclosure provides a method of restoring immune function in a subject suffering from immunosuppression, said method comprising inhibiting the activity of myeloid suppressor cells.

## Brief Description of the Figures

The present invention will be more clearly understood from the detailed description of the preferred embodiments and from the attached figures in which:

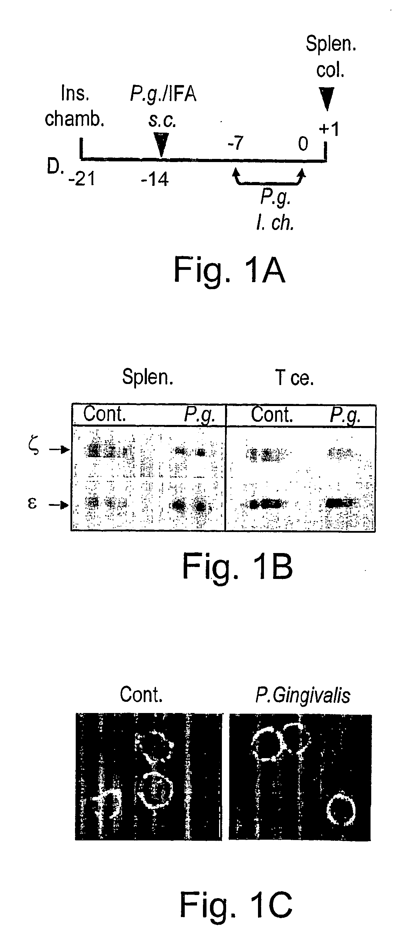

**Figure 1A-F: Treatment with *P. gingivalis* induces specific □ chain downregulation**

**Fig. 1A:** Schematic representation of the experimental model. Normal mice were injected subcutaneously (s.c.) with heat-killed *P. gingivalis* (*P.g.*) in incomplete Freund's Adjuvant (IFA). After 1 and 2 weeks, the mice received intra-chamber (i.ch.) injection of *P.g.* in PBS, and 1 day after the last injection (day +1) the mice were sacrificed. Control mice were injected with PBS instead of *P.g.*

**Fig. 1B:** Equal numbers of control and *P.g.*-treated splenocytes and isolated T cells were lysed and subjected to immunoblot analysis. These were performed with anti-ζ (top panel) and anti-ε (bottom panel).

**Fig. 1C:** Splenocytes isolated from control and *P.g.*-treated mice were analyzed by confocal microscopy. Cells were labeled for surface Thy1.2 with anti-Thy1.2 antibodies (green) and for intracellular ζ with biotinylated anti-ζ antibodies followed by streptavidin-Cy5 (red). The yellow color indicates co-localized signals.

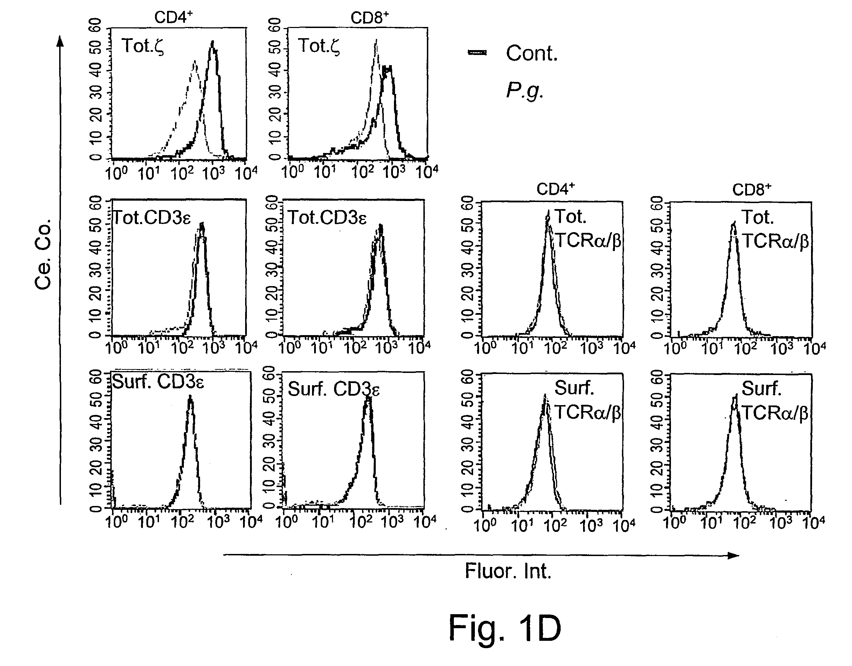

**Fig. 1D:** FACS analysis of the $CD4^+$ and $CD8^+$ cells in the spleen of control and *P. gingivalis*-treated mice. Cells were labeled for surface CD4 and CD8 with PE-labeled anci-CD4 or FITC-labeled anti-CD8 antibodies, respectively. The cells were then stained for surface CD3ε and TCRα/β (bottom panel) using specific biotinylated antibodies followed by streptavidin-Cy5 or fixed, permeabilized and stained for total-ζ chain expression (top panel) or for total CD3ε and TCRα/β expression (middle panel).

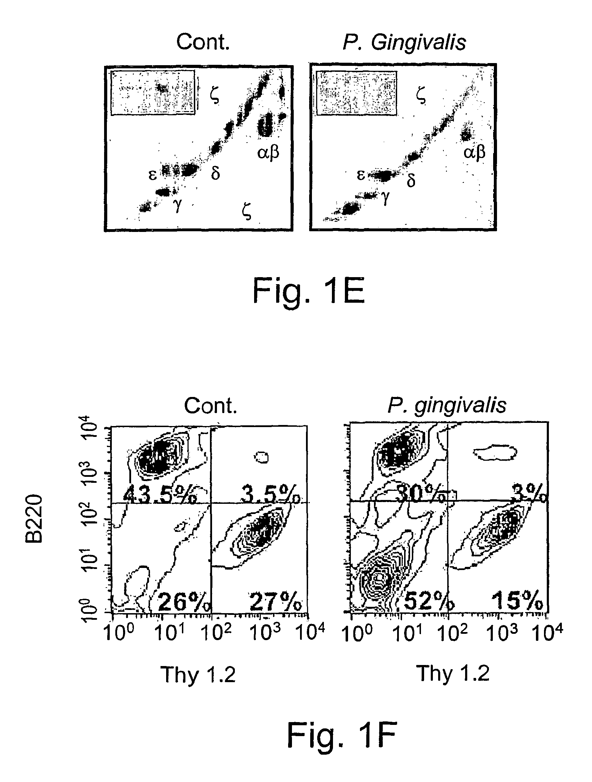

**Fig. 1E:** Splenocytes from control and *P. gingivalis*-treated mice were cell surface biotinylated. The cells were lysed and the TCR was immunoprecipitated with anti-CD3ε and anti-ζ. The immunoprecipitated samples were separated by two-dimensional non-reducing/reducing SDS-PAGE and subjected to immunoblot analysis using streptavidin-HRP. Following longer exposure of the films (insert), the ζ chain was still visualized only in the control group and not in the *P. gingivalis*-treated group.

**Fig. 1F:** T and B cell distribution in the spleen of *P. gingivalis*-treated and control mice. Samples from control and *P. gingivalis*-treated mice were double labeled with anti-Thy 1.2-FITC and PE-labeled anti-B220.

Abbreviations: Ins. chamb., insert chamber; D., days; Splen. col., splenocytes collected; Splen., splenocytes; Cont., control; T ce., T cells; Ce. Co., cell count; Tot., total; Fluor. Int., Fluorescence intensity;

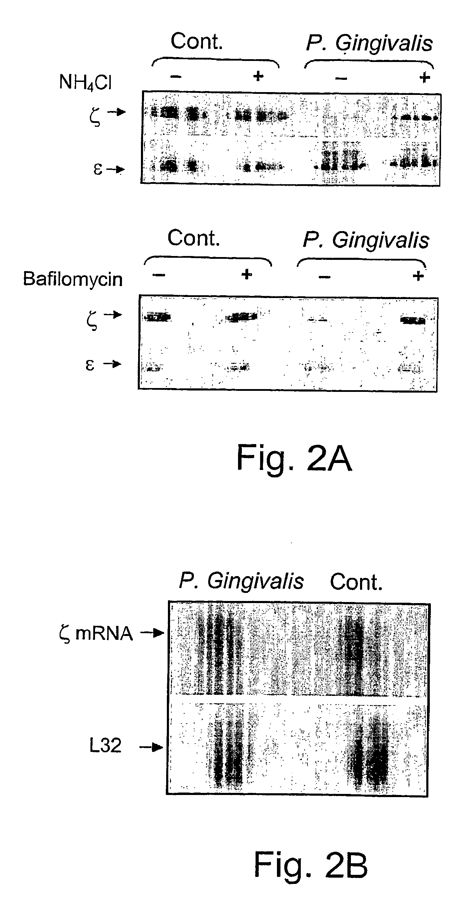

**Figure 2A-B: $\zeta$ downregulation is caused by enhanced lysosomal degradation.**

**Fig. 2A:** Freshly isolated control and *P. gingivalis*-treated splenocytes were incubated with $NH_4Cl$ (20mM) or bafilomycin $A_1$ for 20 and 5 h, respectively at 37°C. Cells were then harvested, washed and lysed. The samples were subjected to immunoblot analysis using anti-$\zeta$ (top panel of each treatment) and anti-CD3$\varepsilon$ (bottom panel of each treatment).

**Fig. 2B:** T cells from the spleen of control and P. gingivalis-treated mice express similar levels of $\zeta$ mRNA. T cells were isolated from the spleens of control and *P. gingivalis*-treated mice, mRNA was prepared, and 20$\mu$g of each sample were subjected to Northern blot analysis. $\zeta$ mRNA levels were detected (top panel) following hybridization with $P^{32}$-labeled $\zeta$-specific cDNA probe. The amount of applied RNA in each group was similar as depicted by the hybridization with $P^{32}$-labeled ribosomal-specific cDNA probe L32 (bottom panel).

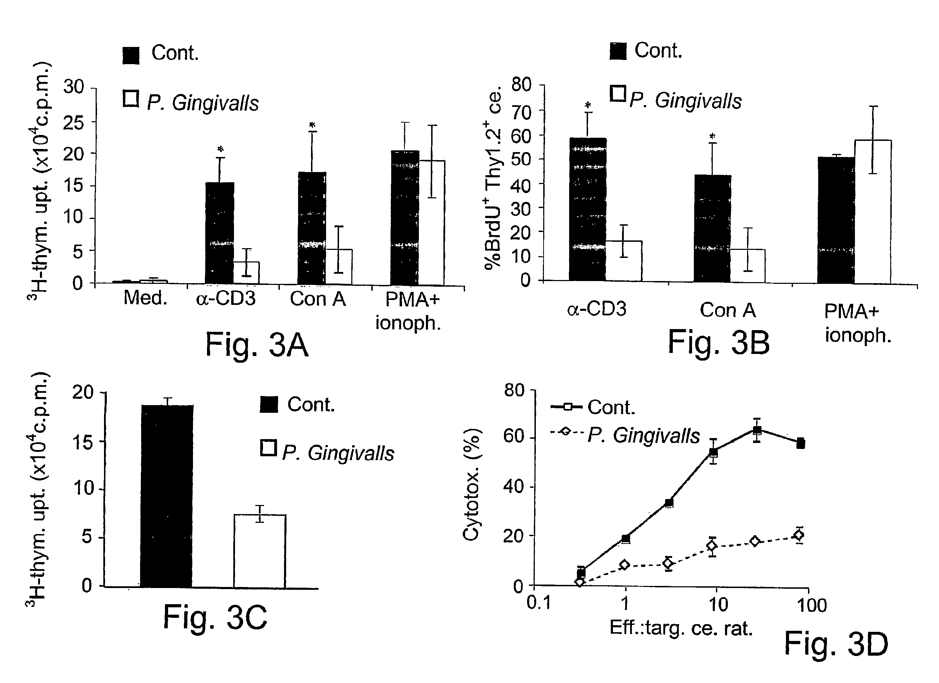

**Figure 3A-D: T cells isolated from P. gingivalis-treated mice show impaired immune function.**

**Fig. 3A:** Splenocytes from control and *P. gingivalis*-treated mice were activated with anti-CD3 antibodies, Con A, a combination of PMA and $Ca^{2+}$ ionophore or medium and then, cell proliferation was measured as $^3H$-thimidine uptake. Results are presented as the mean value of triplicate cultures.

**Fig. 3B:** Splenocytes from control and *P. gingivalis*-treated mice were activated as in (a) and specific T cell proliferation was measured by flow cytometry by BrdU incorporation in Thy 1.2 positive cells.

In Fig. 3A and 3B the results are presented as the mean value of three independent experiments and the standard deviations are shown. The cells from the P. gingivalis-treated mice showed a significantly lower response to stimulation with anti-CD3 and Con A when compared with the cells from the control mice, as determined by the Student's t-test

(* p <0.001 for (a), p< 0.002 for (b)).

**Fig. 3C:** MLR response of T cells in the spleen of control and *P. gingivalis*-treated mice. Splenocytes were co-cultured with equal numbers of irradiated allogeneic splenocytes for 3 days and then, $^3H$-thimidine uptake was measured.

**Fig. 3D:** Cell-mediated cytotoxicity was assayed in splenocytes isolated from control and *P. gingivalis*-treated mice as described in the Experimental Procedures. Percent cytotoxicity was calculated as indicated. Results are presented as the mean cytotoxicity value of triplicate cultures as a function of effector-target cell ratio.

Abbreviations: Cont., control; $^3H$-thym. upt., $^3H$-thymidine uptake; Med., medium; ionoph., ionophore; ce., cells; Cytotox., cytotoxicity; Eff.:targ. ce. rat., Effector:target cell ratio.

**Figure 4A-B: *P. gingivalis*-treated mice have impaired ability to clear an influenza infection.**

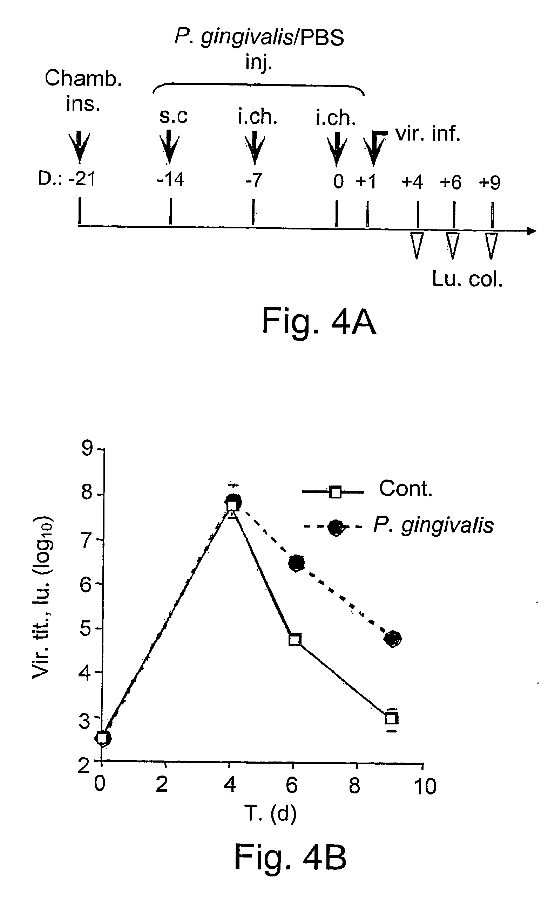

**Fig. 4A:** Flow chart of *P. gingivalis* (*P.g.*) treatment and influenza virus infection. Mice were infected with influenza virus after the *P.g.* or PBS injections (sub-cutaneous (s.c.) and intra-chamber (i.ch.)) were completed, as described in Fig. 1a. At various time points following the infection, lungs were collected and analyzed for virus titers.

**Fig. 4B:** Lungs from control and *P. gingivalis-treated* mice were extracted at the noted days after virus inoculation, and influenza virus titers were determined. The results presented are the means of two independent experiments. Standard deviation bars are indicated.

Abbreviations: Chamb. ins., Chamber insertion; D., days; inj., injection; vir. inf., virus infection; Lu. col., Lungs collected; Cont., control; Vir. tit., virus, titer; Lu., lung; T., time.

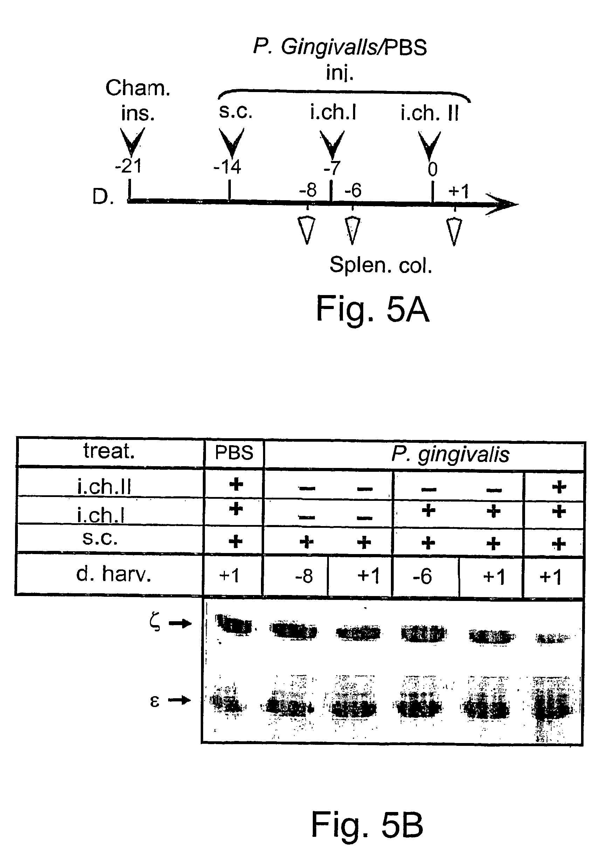

**Figure 5A-B: Sustained exposure to antigen is necessary to induce $\zeta$ downregulation.**

**Fig. 5A:** Schematic representation of the experimental model. The time points of splenocyte harvest are indicated.

**Fig. 5B:** Splenocytes isolated from control mice (first lane) or mice subjected to different numbers of *P. gingivalis* (*P.g.*) injections were harvested at the indicated time points, lysed and subjected to immunoblot analysis using anti-$\zeta$ and anti CD3$\varepsilon$. Subcutaneous (s.c.), the first (i.ch. I) and second (i.ch. II) intra-chamber *P.g.* or PBS injections are indicated.

Abbreviations: Chamb. ins., Chamber insertion; D., days; inj., injection; Splen. col., splenocyte collection; Treat., treatment; d. harv., day harvested.

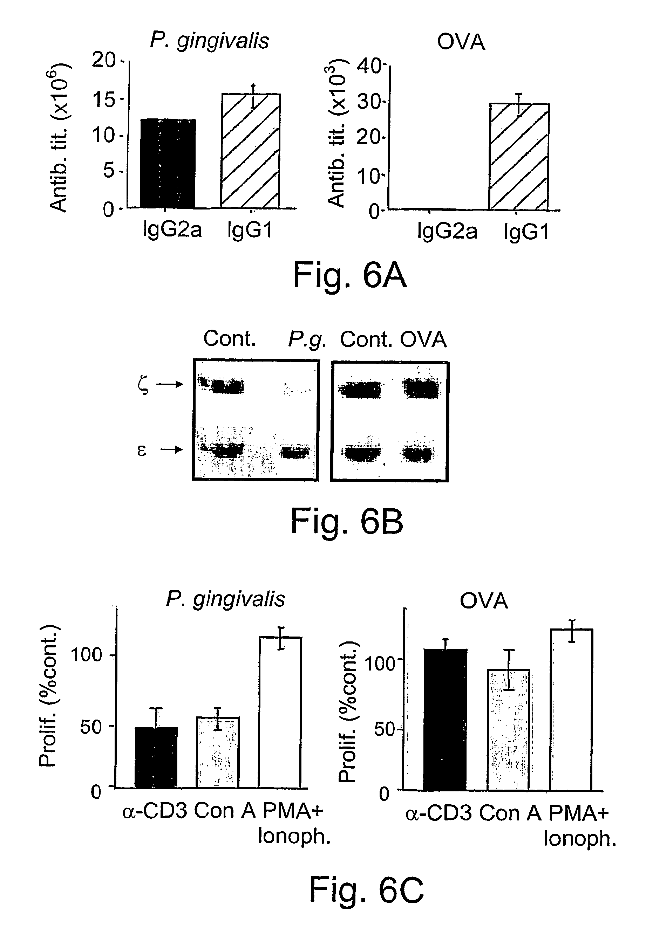

**Figure 6A-B: A $T_H1$ immune response is necessary for the induction of $\zeta$ chain downregulation and T cell dysfunction.**

Mice were treated with *P. gingivalis (P.g.)* and IFA or with OVA and Alum using the same regimen depicted in Fig. 1A.

**Fig. 6A:** To determine the type of response, mice were bled at day +9 of the experiment and the titer and isotype of the specific antibodies was established by ELISA. Endpoint titers are expressed as the reciprocal values of the last dilution with an OD of 0.1 at 405 nm above maximal absorbance values of samples collected from non-immunized mice.

**Fig. 6B:** Splenocytes from mice treated with both regimens were analyzed by immunoblotting using anti-ζ (upper panels) and anti-ε (lower panels). (c) The proliferative response of T cells isolated from *P.g.* or OVA-treated mice was measured as depicted in Fig. 3a. Results are presented as percent of the response measured in control mice following each treatment.

Abbreviations: Antib. tit., antibody titer; Cont., control; Prolif., proliferation; ionoph., ionophore.

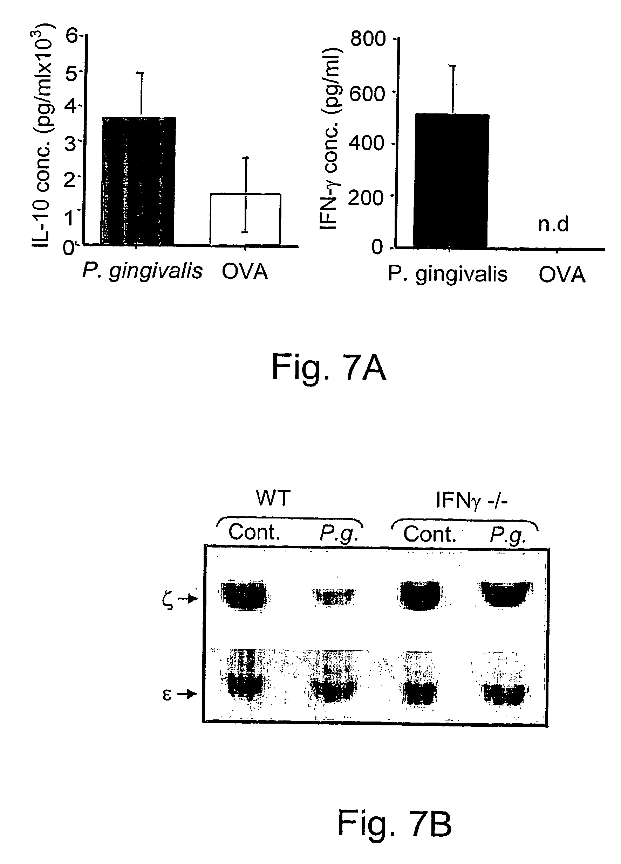

**Figure 7A-C: IFN-γ mediates the induction of ζ chain downregulation.**

**Fig. 7A:** Cytokine concentrations were determined in the chamber exudates obtained from individual mice treated with either *P. gingivalis* (*P.g.*) and IFA or OVA and Alum by ELISA. The results are presented as the mean value of the individual mice in each treatment group. (n.d.) not detectable values of IFN-γ in the OVA-treated mice.

**Fig. 7B:** Immunoblot analysis of splenocytes isolated from wild-type or IFN-γ deficient mice (IFNγ -/-) that were treated with *P.g.* as depicted in Fig. 1a.

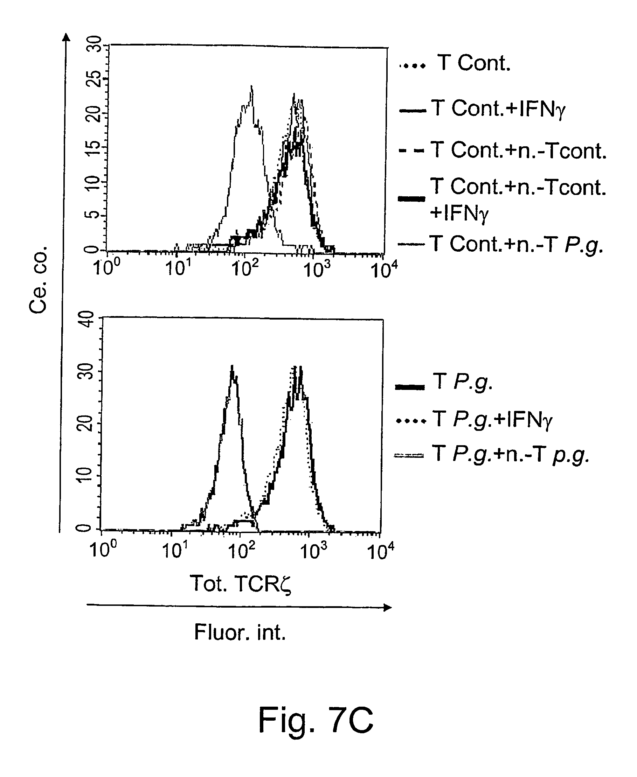

**Fig. 7C:** T cells from control (upper panel) and *P.g.*-treated mice (lower panel) were isolated from the spleen using FITC labeled anti-Thy1.2 and anti-FITC conjugated magnetic beads. T cells alone or mixed with the indicated non-T cell populations were incubated in *vitro* for 24 h in the presence or absence of IFN-γ (50 U/ml). Cells were stained for cell surface Thy 1.2 and for total ζ chain expression, as described in Fig. 1A-F, and ζ chain expression is shown.

Abbreviations: conc., concentration; wt, wild type; Cont., control; n.-T, non-T; Ce. co., cell count; Fluor. Int., Fluorescence intensity.

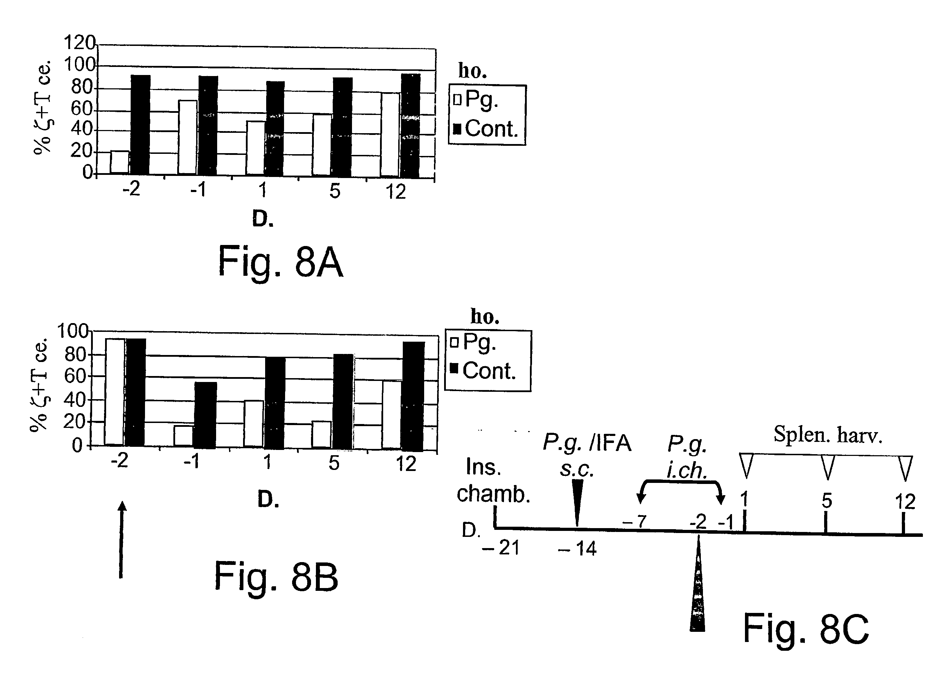

**Figure 8A-C: Normal T cells downregulate ζ expression when injected into the *P.-gingivalis*-treated mice.**

**Fig. 8A:** Histogram showing the level of ζ chain in the host cells within the spleen.

**Fig. 8B:** Histogram showing the level of ζ chain in the donor cells within the spleen of the host animal. Donor, PKH-labeled normal cells were injected on day -2, as indicated by the arrow.

**Fig. 8C:** Tine-line of the experiment. At day -2, as indicated by the long arrowhead, PKH-labeled normal splenocytes were injected into the spleen.

Abbreviations: T ce.; T cells; ho. hosts; s.c., subcutaneous injection; i.ch., intrachamber injection, Ins. chamb., Insert chamber; Splen. harv. Splenocyte harvest.

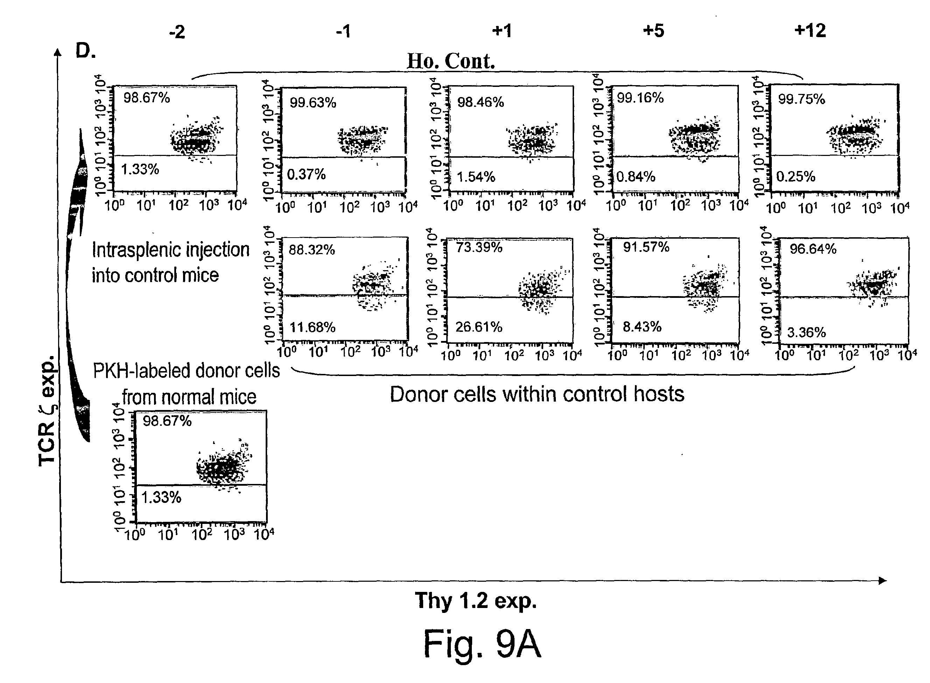

**Figure 9A-B: Normal T cells downregulate ζ expression when injected into the *P. gingivalis*-treated mice.**

These Figures present the same results as in Fig. 8, but the data is presented as dot-blot FACS analysis. The horizontal line in each quadrate is designated according to the background fluorescence of each sample. The indicated numbers represent the % of ζ+ T cells of the total PKH-labeled T cells (donor) or non-labeled T cells (hosts) in each spleen.

**Fig. 9A:** PKH-labeled splenocytes from normal mice (bottom left, day -2) were injected into control hosts (top left, day -2)

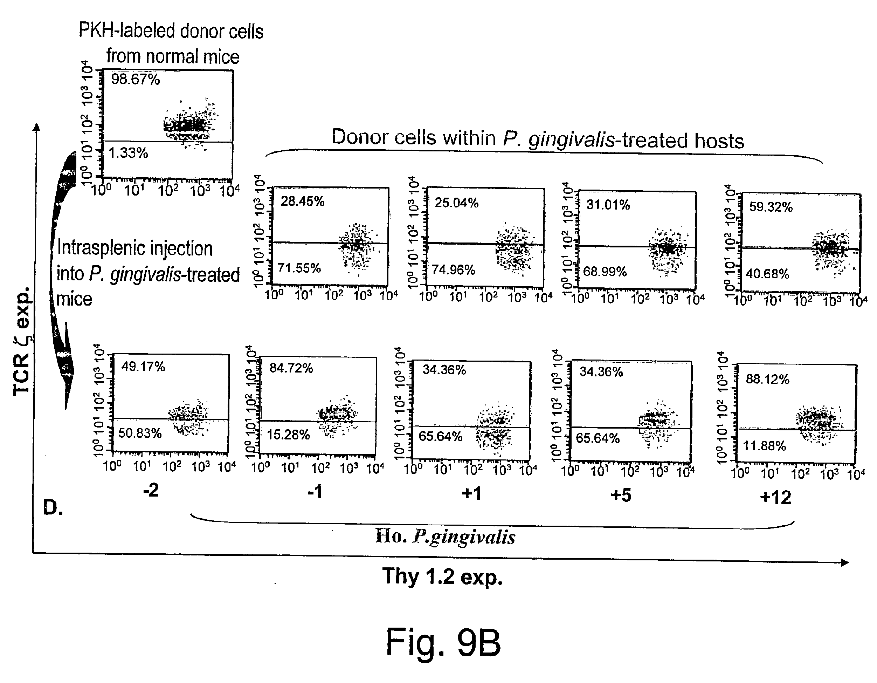

**Fig. 9B:** PKH-labeled splenocytes from normal mice (top left, day -2) were injected and into P. gingivalis-treated hosts (bottom, day -2).

At different time points spleens were harvested and ζ expression was analyzed in the hosts' T cells (controls in blue; *P. gingivalis-treated* in green) and in the donor T cells localized in the spleen of the hosts (donor cells within the controls in red squares below the blue; donor cells within *P. gingivalis*-treated hosts in red squares above the green).

Abbreviations: D., day; Ho., Host; exp., expression.

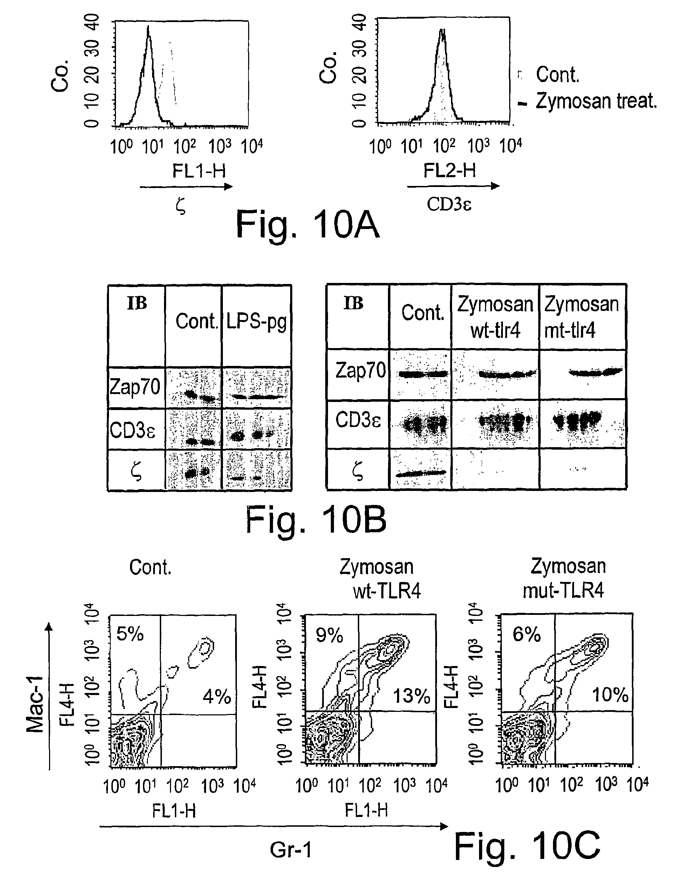

**Figure 10A-E: Treatment with TLR-2 ligands induces specific ζ chain down-regulation and impaired function.**

**Fig. 10A:** FACS analysis histograms that represent the total expression of ζ-chain and CD3ε of gated splenic T cells (Thy1.2+ cells) from zymosan-treated mice (black) compared to control mice, injected with adjuvant and OVA (gray).

**Fig. 10B:** Immunoblot (IB) with anti-Zap70, anti-CD3ε and anti-ζ antibodies of splenic T cells from wt-TLR4 and mut-TLR4 mice, treated with LPS-pg or zymosan, compared to control mice.

**Fig. 10C:** Gr1+ and Mac1+ cells distribution in the spleen of zymosan-treated and control mice. Numbers in quadrants indicate the percentage of a specific cell population of the total splenocytes.

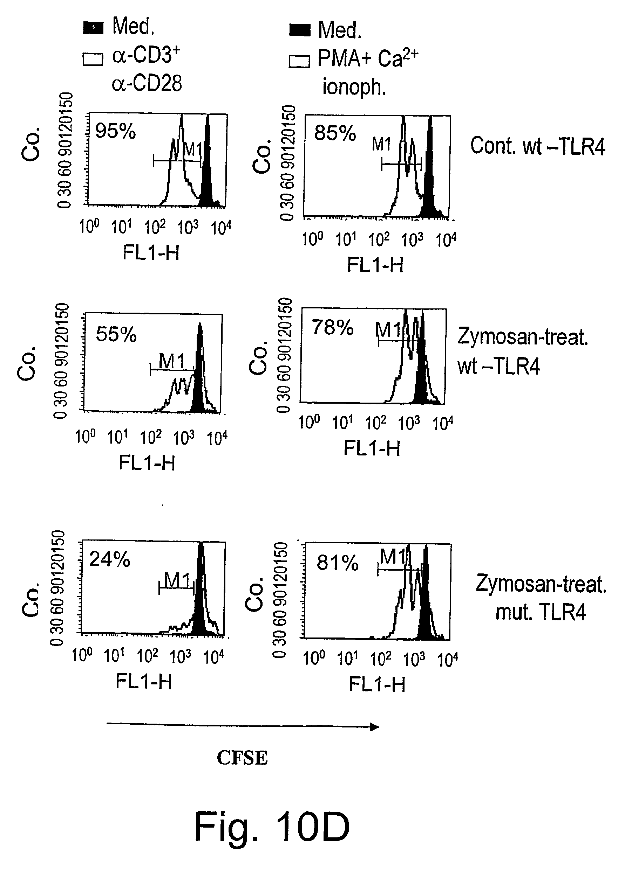

**Fig. 10D:** A lower percentage of CFSE-labeled splenic T cells, that divided 1-4 times, following 48 hours activation with anti-CD3 and anti-CD28, in zymosan-treated mice (wt-TLR4 and mutTLR4) relative to splenic T cells from control mice (55%, 24%, 95%, respectively). Activation with PMA and ionophore, that activate signaling pathways downstream to the TCR, partially overcame this impaired function (78%, 81%, and 85%, respectively).

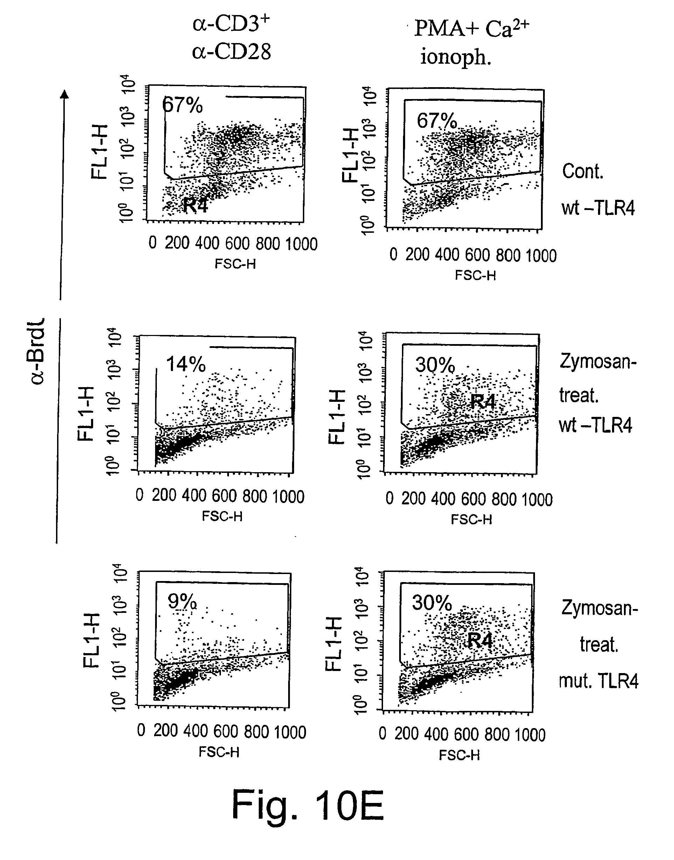

**Fig. 10E:** FACS analysis showing the percentage of BrdU+ Thy1.2+ cells in total splenic T cells from control mice relative to splenic T cells from zymosan treated mice (wt-TLR4 and mut-TLR4) (67%, 14%, 9%, respectively). While activation with PMA and ionophore could partially overcome this impaired function (67%, 30%, 30%, respectively).

<u>Abbreviations:</u> Cont., control; treat., treated; Med., medium; mut., mutant; Co., counts; ionoph., ionophore.

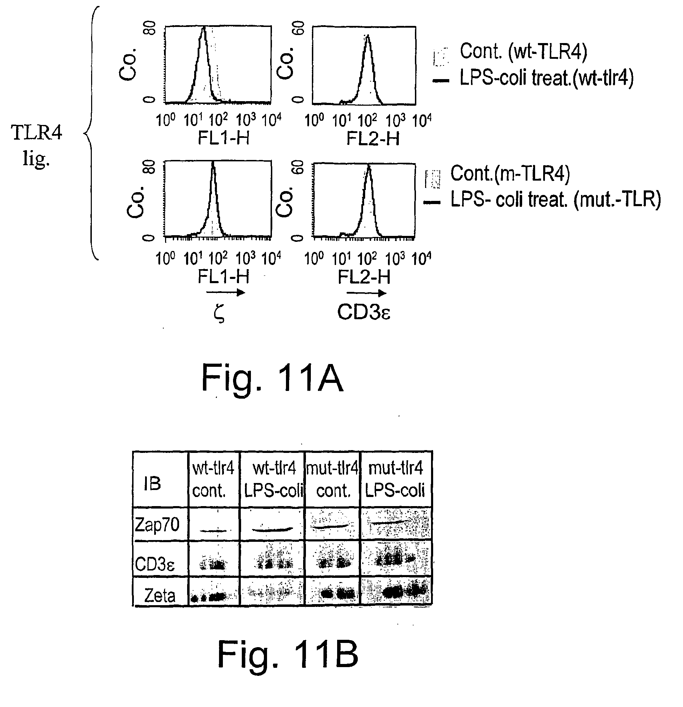

**Figure 11A-E: Treatment with TLR4 ligand induces specific $\zeta$ chain down-regulation and impaired T cell proliferation.**

**Fig. 11A:** FACS analysis histograms showing the total expression of $\zeta$-chain and CD3$\epsilon$ of gated splenic T cells (Thy1.2+ cells) from LPS from *E.coli*-treated mice [C3H/Hen (wt-tlr4)] (black) compared to splenic T cells from control mice [C3H/Hen (wt-tlr4)] injected with adjuvant and OVA (gray) and TLR4 mutant mice [C3H/Hej(mutant th-4)].

**Fig. 11B:** Immunoblot (IB) with anti-Zap70, anti-CD3$\epsilon$ and anti-$\zeta$ of splenic T cells from LPS-coli treated mice, control mice, and TLR-4 mutated mice [C3H/Hej (mutant tlr4)].

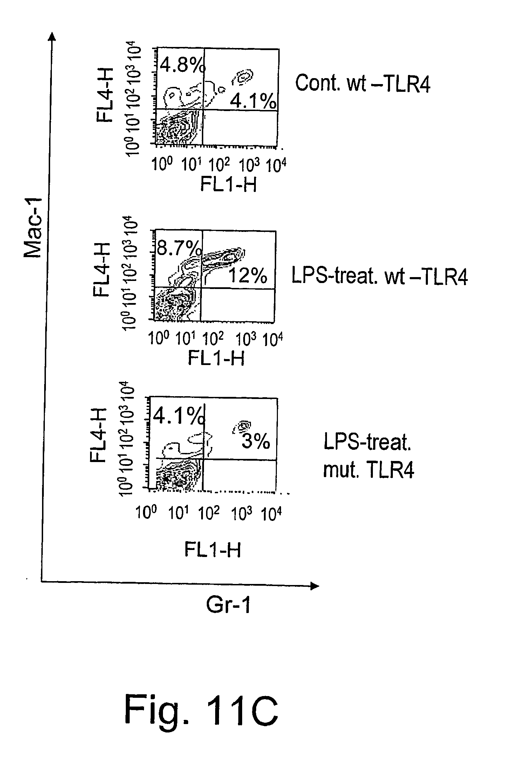

**Fig. 11C:** Gr1+[and] Mac1+ cells distribution in the spleen of LPS-treated and non-treated mice, both from wild type (wt-tlr4) and mutant mice (mutant tlr4). Numbers in quadrants indicate the percentage of a specific cell population of the total splenocytes.

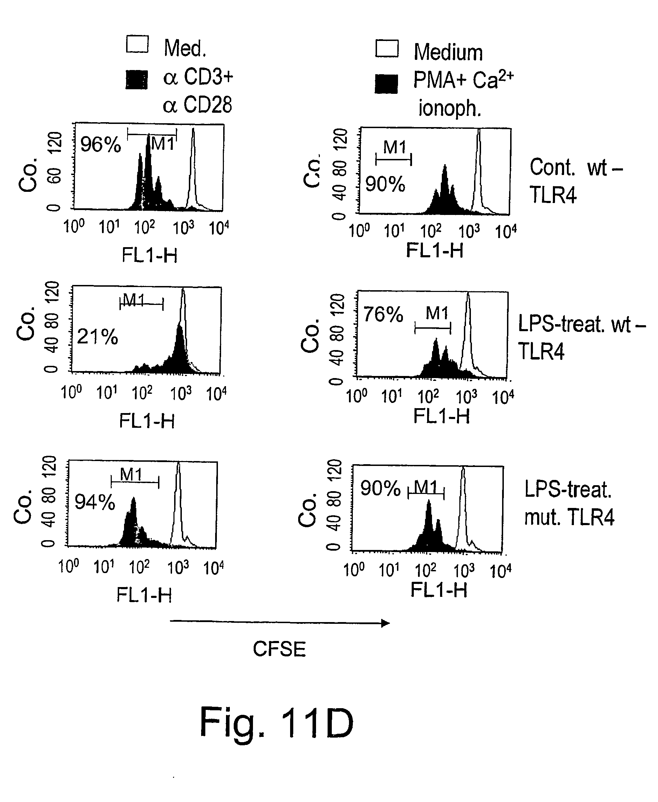

**Fig. 11D:** CFSE-labeled splenocytes were activated with anti-CD3$\epsilon$ and anti-CD28 (left column) or with combination of PMA and calcium ionophore (right column) for 60-72 hours. A lower percentage of CFSE-labeled splenic T cells from LPS-*E. coli* treated mice [C3H/Hen(wt-tlr4)], which divided 2-5 times following activation with anti-CD3 and anti-CD28, relative to splenic T cells from control mice or TLR4 mutated mice [C3H/Hej(mutant tlr4)](21%, 96%, 94%, respectively). While activation with PMA and ionophore could overcome this impaired function (76%, 90%, 90%, respectively).

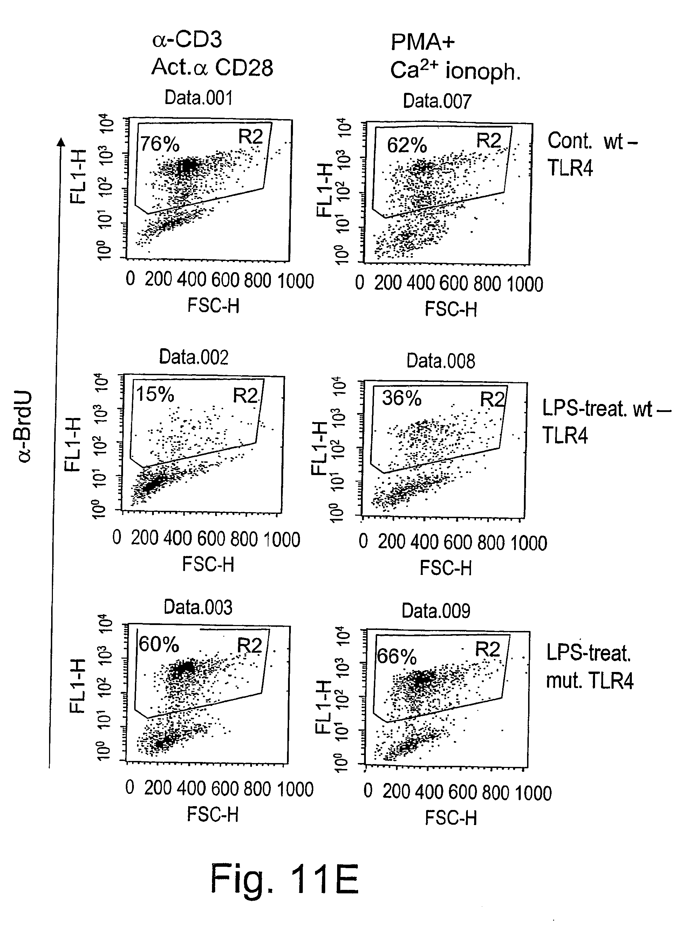

**Fig. 11E:** FACS analysis representing the percentage of BrdU+ Thy1.2+ cells in total splenic T cells from LPS-coli treated mice relative to splenic T cells from control mice or TLR4 mutated mice (15%, 76%, and 60%, respectively).

<u>Abbreviations:</u> lig., ligand; cont., control; treat., treated; mut., mutant; med., medium; Act., activators; Co., counts; ionoph., ionophore.

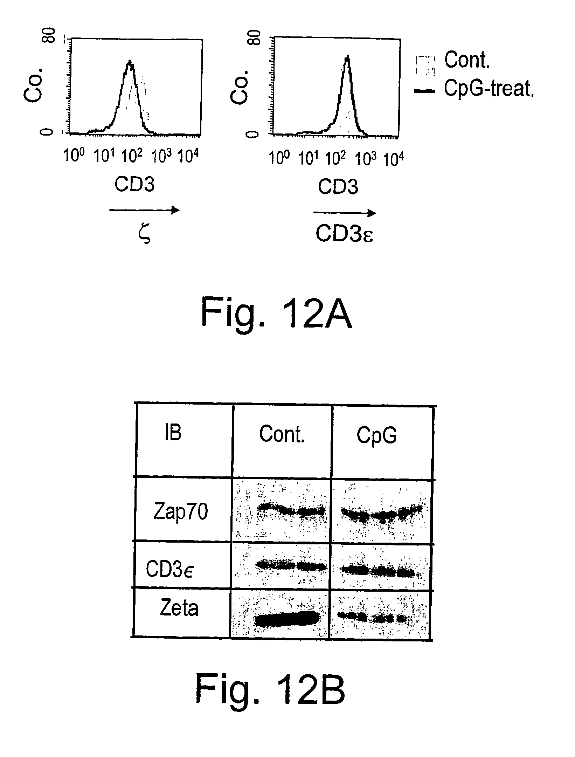

**Figure 12A-E: Treatment with TLR9 ligand induces specific $\zeta$ chain down-regulation and Impaired T cell proliferation.**

**Fig. 12A:** Histograms of FACS analysis representing the total expression of $\zeta$-chain and CD3$\epsilon$ of gated splenic T cells (Thy1.2+ cells) from TLR9 ligand (CpG-ODNs) treated mice (BALB/c) (black), compared to splenic T cells from control mice, which were injected with mutated CpG-ODNs (gray).

**Fig. 12B:** Immunoblot (IB) with anti-Zap70, anti-CD3$\epsilon$ and anti-$\zeta$ of splenic T cells from CpG-ODNs treated mice compared to control mice (injected with mutated CpG-ODN).

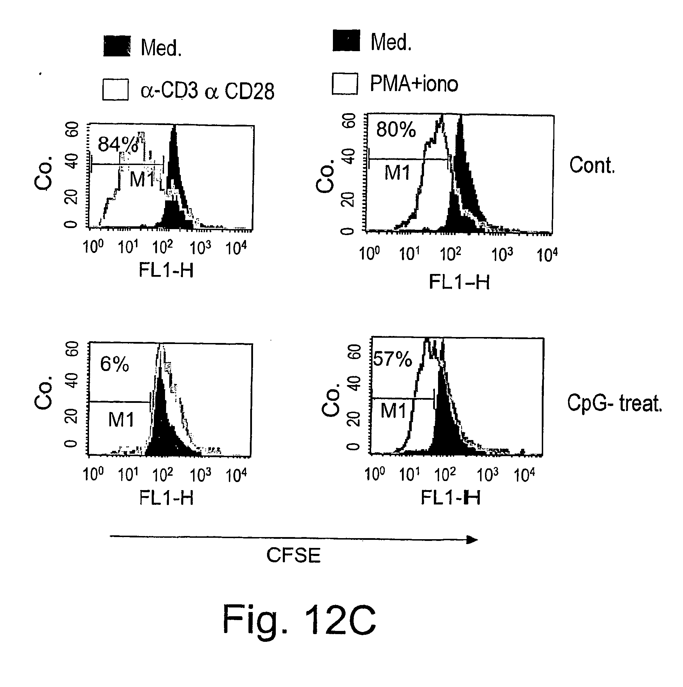

**Fig. 12C:** CFSE-labeled splenocytes were activated with anti-CD3$\epsilon$ and anti-CD28 (left column) or with combination of PMA and calcium ionophore (right column) for 60 hours. A lower percentage of CFSE-labeled splenic T cells, which divided 1-5 times, following activation with anti-CD3 and anti-CD28, from CpG-treated mice relative to control mice (6%, 84%, respectively). While activation with PMA and ionophore could overcome this impaired function (57%, 80%, respectively).

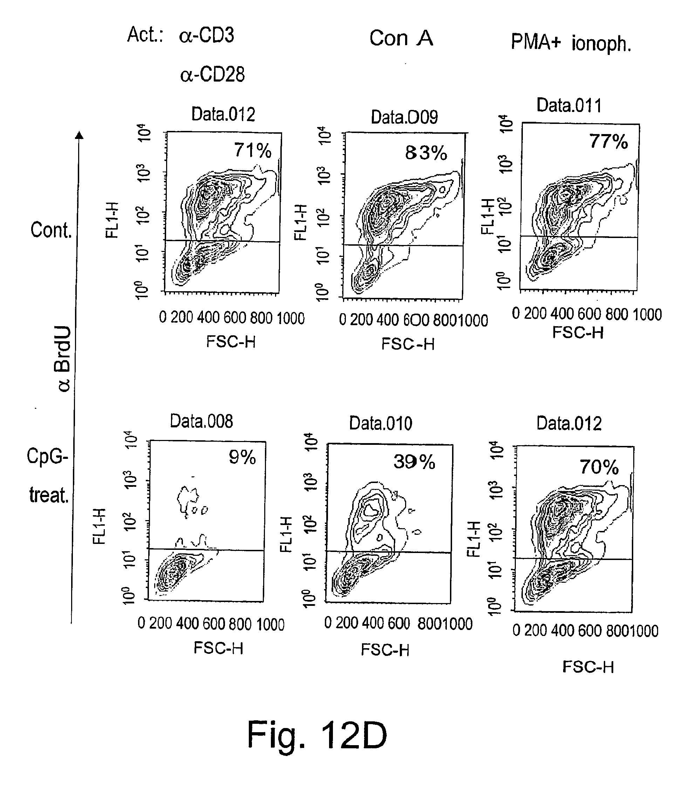

**Fig. 12D:** FACS data presenting BrdU incorporation in Thy-1.2+ spleen cells. A lower percentage of BrdU+ T cells of total T cells from CpG-treated mice relative to control mice following activation with anti-CD3 and anti-CD28 (9%, 71%, respectively) $\zeta$ or with ConA (39%, 83%, respectively). While activation with PMA and ionophore could overcome this impaired function (80%, 57%, respectively).

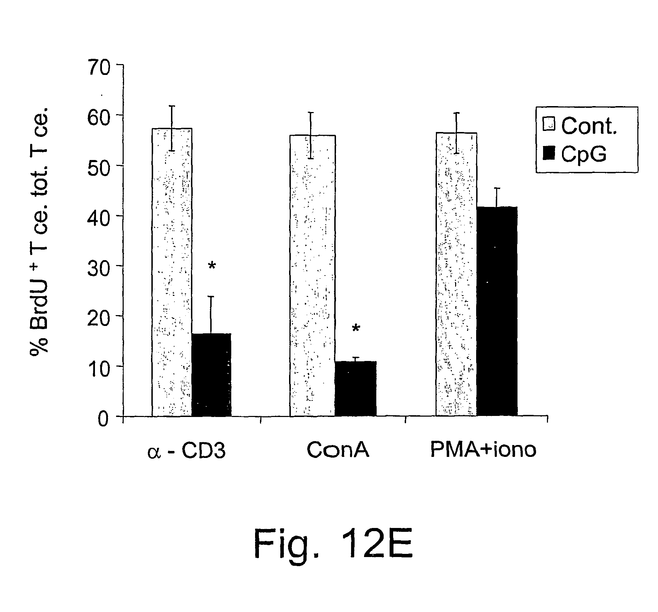

**Fig. 12E:** The results are presented as the mean value of three independent experiments, and standard deviations are shown. T cells from CpG-treated mice show a significantly lower response to stimulation with anti-CD3 and Con A than do cells from control mice: *, $P < 0.002$ (Student's t-test).

<u>Abbreviations:</u> Cont., control; treat., treated; Act., activators; Med., medium; ce., cells; tot., total; ionoph., ionophore.

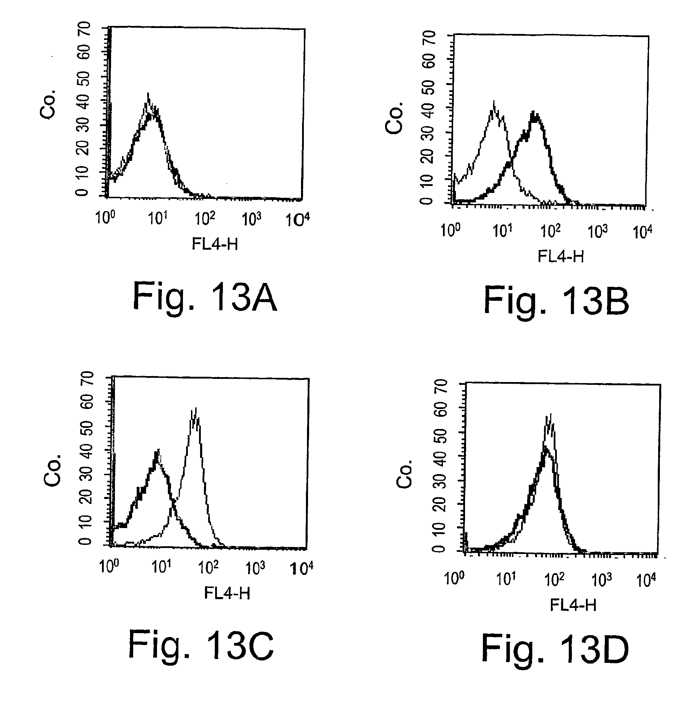

**Figure 13A-D: Co-incubation of T cells and Gr-1+ Mac-1+ enriched population.**

T cells from *P.g.*-treated and control mice were isolated and incubated together with Gr-1+/Mac-1+ (double positive) enriched population. The levels of the TCR $\zeta$ chain expression were analyzed by FACS, before (thinner line) and after (thicker line) 24 hours incubation.

**Fig. 13A:** T cells from *P.g.*-treated mice exposed to Gr-1+/Mac-1+ enriched population from *P.g.* mice retain the downregulation of $\zeta$ chain expression.

**Fig. 13B:** T cells from *P.g.*-treated mice exposed to Gr-1+/Mac-1+ enriched population from control mice recover the levels of $\zeta$ chain expression.

**Fig. 13C:** T cells from control mice exposed to Gr-1+/Mac-1+ enriched population from *P.g.*-treated mice downregulate ζ chain expression.

**Fig. 13D:** T cells from control mice exposed to Gr-1+/Mac-1+ enriched population from control mice retain normal levels of ζ chain expression.

Abbreviations: Co., counts.

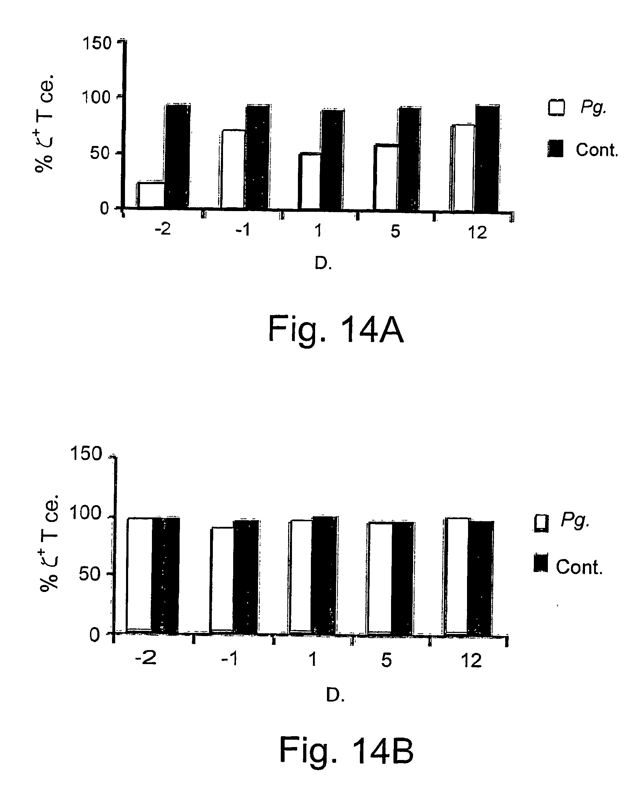

**Figure 14A-D: Expression of ζ chain in T cells from lymph nodes and spleen from *P.gingivalis*-treated and non-treated mice injected with splenocytes from non-treated mice.**

**Fig. 14A:** Level of ζ chain expression in spleen cells from the host.

**Fig. 14B:** Level of ζ chain expression in lymph node cells from the host.

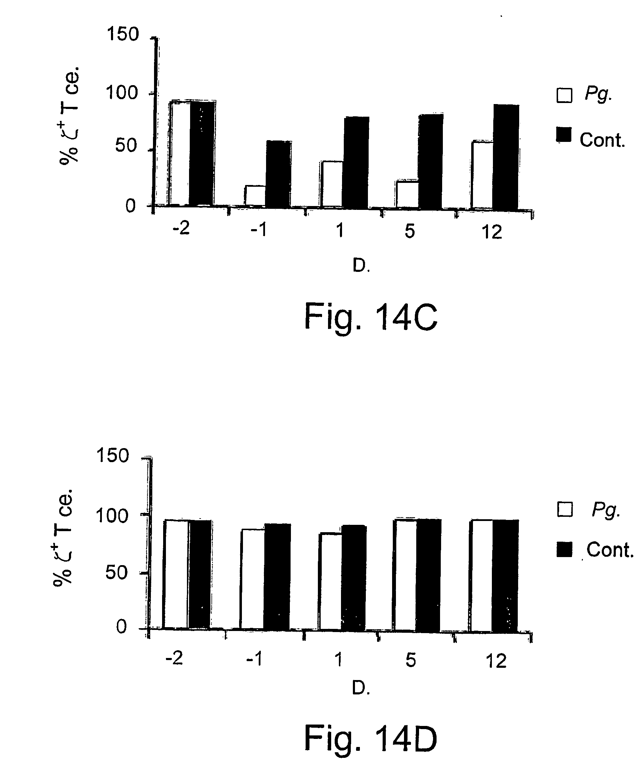

**Fig. 14C:** Level of ζ chain expression in spleen cells from the donor.

**Fig. 14D:** Level of ζ chain expression in lymph node cells from the donor.

Abbreviations: T ce., T cells; cont., control; D., day.

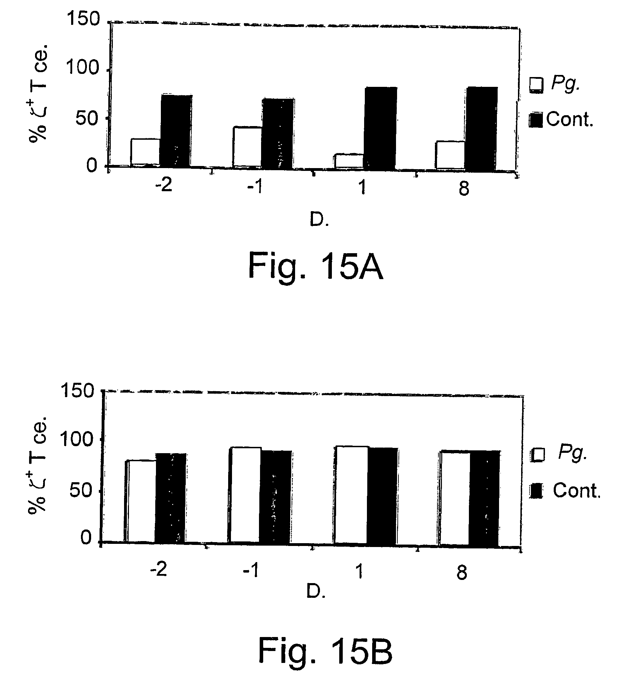

**Figure 15A-D: Expression of ζ chain in T cells from lymph nodes and spleen from *P.gingivalis*-treated and non-treated mice injected with splenocytes from *P.gingivalis*-treated mice.**

**Fig. 15A:** Level of ζ chain expression in spleen cells from the host.

**Fig. 15B:** Level of ζ chain expression in lymph node cells from the host.

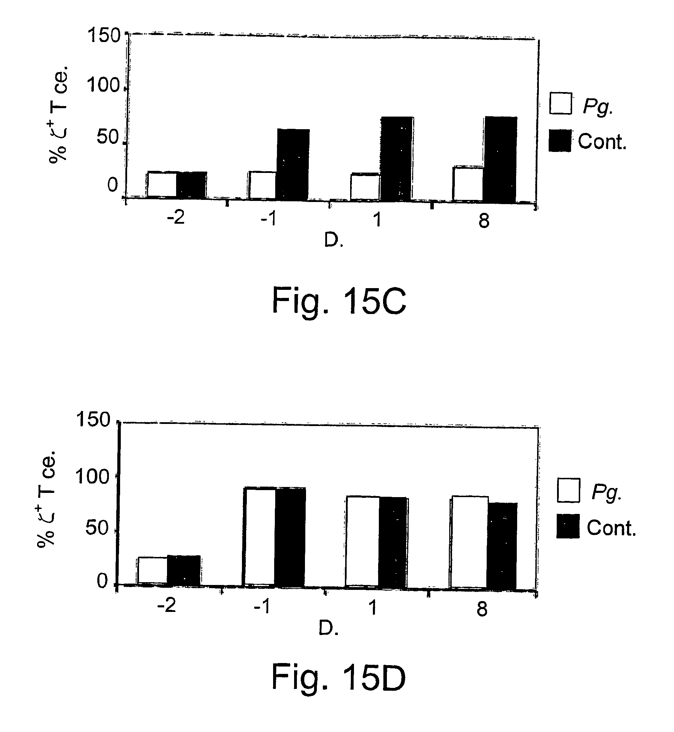

**Fig. 15C:** Level of ζ chain expression in spleen cells from the donor.

**Fig. 15D:** Level of ζ chain expression in lymph node cells from the donor.

Abbreviations: T ce., T cells; cont., control; D., day.

## Detailed Description of the Invention

The following abbreviations are employed throughout this specification:

- APC: Antigen presenting cell
- ARG 1: arginase 1
- CFA: complete Freund's Adjuvant
- CFSE: 5- and 6-carboxyfluorescein diacetate succinimidyl ester
- ConA: Concanavalin A
- CTL: cytotoxic T lymphocytes
- FITC: fluorescein isothyocyanate
- i. ch.: intra-chamber
- IFA: incomplete Freund's Adjuvant
- IFNγ: interferon γ
- IL-10: interleukin 10
- LPS: lipopolysacharide
- MLR: mixed leukocyte reaction
- MSC: myeloid suppressor cells
- NOS2: nitric oxide synthase
- ODN: oligodeoxynucleotide
- OVA: ovalbumin
- PE: phycoerythrin
- *P.g.: P. gingivalis*
- PMA: phorbol 12-myristate 13-acetate
- s.c.: sub-cutaneous
- SPF: sterile pathogen-free
- TCR: T cell receptor
- TLR: Toll-like receptor

In the present study, the inventors describe an *in vivo* model or experimental system, which mimics a situation of chronic systemic inflammation, wherein the exposure of animals to antigens causes T cell antigen receptor (TCR) ζ chain downregulation.

In the herein described experimental model, healthy mice were repeatedly exposed to antigens, inducing a $T_H1$-dominant inflammatory immune response, which was IFNγ dependent, and was mostly evidenced by the downregulation of TCRζ chain expression and impairment of T cell function, measured by reduced proliferation following various stimuli which require TCR integrity.

Interestingly the inventors show that the model system may be generated upon stimulation with a wide range of antigens, such as heat-killed Gram negative bacteria (e.g. *P. gingivalis*), heat-killed Gram positive bacteria (e.g. *Streptococcus mutans*), inactivated influenza virus, heat-killed mycobacteria, Toll-like receptor (TLR) ligands, and others.

Thus, in a first aspect, the present disclosure refers to a model system for TCR ζ downregulation, wherein said model system is an animal model, preferably mouse, and wherein said animal has been injected with an antigen, said antigen being preferably in admixture with an adjuvant which induces an inflammatory immune response. The model system of the invention may be for *in vivo* or *ex vivo* use.

The system herein presented offers a number of advantages. The use of healthy mice and heat-killed bacteria or an antigen-adjuvant combination, enabled the inventors to avoid any physiological and pathological symptoms typical to a particular disease that might interfere with the results. The antigens, delivered either through the pre-implanted chambers, or via subcutaneous or intrafootpad injections, in combination or not with intraperitoneal injections, were slowly released and induced local and systemic inflammatory immune response, thus mimicking a chronic pathology. In addition, the local and time-limited exposure to the antigens enabled the inventors to test how this treatment affects ζ chain expression and T cell function in distal secondary lymphatic organs, particularly in the spleen, during the chronic exposure to the antigen and following antigen withdrawal. Last but not least, in the chamber system, secreted cytokines accumulate in the chamber, making sampling easy at various time points.

It should be noted that the terms T cells and T lymphocytes are used interchangeably throughout this application.

As shown in the following Examples, the simple exposure of normal, healthy mice to antigens that induce systemic inflammation can induce dramatic TCR ζ chain downregulation in T lymphocytes, particularly T lymphocytes from spleen and peripheral blood of these mice. Moreover, the inventors found that interferon gamma (IFNγ), a Th1-cytokine, plays a key role in the induction of this phenomenon. Among the TCR subunits, only expression of the ζ chain was affected. T cells isolated from the treated, i.e., injected mice showed impaired *in vitro* and *in vivo* immune functions. All these results account for a suppressive environment, which affects the T cells and induces ζ downregulation and impaired T cell function in the treated mice.

It is to be understood that TCR ζ chain downregulation was evidenced by diminished detection of the protein, as demonstrated, *inter alia*, in Figure 1B, indicating that lower amounts of the protein were present in the cells tested. In addition, the inventors demonstrate that this ζ chain downregulation is due to its targeting to lysosomal degradation (see Example 2 and Fig. 2A-B).

Thus, in an aspect of the present disclosure, the TCR ζ downregulation in the model system is associated with the following properties: (a) it is IFNγ-dependent; (b) it requires sustained exposure to the antigen; (c) it requires the development of a TH1-dependent inflammatory immune response.

Sustained exposure to the antigen refers to chronic exposure. As shown in the Examples, the mice were continuously exposed to an antigen throughout a period of at least two weeks (14 days) to three weeks, or possibly more.

Interestingly, the inventors also observed that ζ chain expression was not affected in T cells from the lymph nodes of antigen-treated mice, as shown in Example 11 and Figs. 14A-D and 15A-D. This is in contrast to the dramatic downregulation of ζ chain expression in the T cells isolated from the peripheral blood or spleen. Thus, these results show that two environments are created in the created animals. One damages the T cells and induces ζ chain downregulation (in peripheral blood and spleen). The second does not affect ζ chain expression levels (in lymph nodes), which remain normal. One explanation for these two environments is that the main difference between them resides in the suppressor cells, related to the myeloid lineage. While myeloid suppressor cells are recruited to the spleen and periphery, they are absent from the lymph nodes. The inventors showed that T cells in the spleen, expressing low ζ chain levels, recover in the lymph nodes. However, although expression levels of the TCR ζ chain are normal in the lymph nodes of the treated mice, its low levels in the periphery and spleen determine the immune status of the treated animals. Hence, the general immune status is impaired, in the animal model system, in regards to T cell-dependent immune response, as shown in Example 4, by the slow rate of influenza virus clearance when compared to non-treated mice.

In this *in vivo* model system, ζ chain downregulation and impaired *in vivo* and *in vitro* T cell function was induced, in a manner similar to that observed in a wide range of pathologies. ζ chain downregulation and impaired *in vitro* T cell function correlated with an impaired *in vivo* T cell-mediated immune response. This phenomenon required IFN-γ, sustained exposure to antigen and a TH1-dependent inflammatory immune response.

The animal model system of the disclosure could therefore be used as a tool for testing substances that could potentially block such phenomenon. A test substance could affect the T cells themselves and operate within these cells increasing ζ chain expression. Alternatively, a test substance could interfere with the non-T cells, or neutralize a putative secreted factor responsible for triggering the "sick", harmful environment generated in the injected animals. Hence, in a third aspect, the model system of the disclosure is valuable for the screening of substances that upregulate or prevent the downregulation of the TCR ζ chain.

In a further aspect, the model system of the disclosure may be applied for assessing the feasibility of immunotherapies, i.e., for evaluating the sensitivity of cells, which are to be used in immunotherapy, to the environment of the model system.

For example, T cells engineered to express single chain antibodies against specific antigens may be tested for their viability and activity in an immunosuppressed environment, as the one generated in the model system. This immunosuppressed environment could also negatively affect the efficiency of the immune response when a vaccination is given to the affected mice in the model system.

Example 8 demonstrates how the environment generated in the model system is harmful for the functioning of normal T cells, since injection of T cells obtained from healthy mice (which would be the equivalent of cells aimed for immunotherapy) into the mouse model (the affected hosts, or the sick subject) induced TCR ζ chain downregulation. This result indicates that the harmful environment affects not only the host's T cells but also the donor's cells. Similar results were observed when T cells obtained from healthy mice were exposed *in vitro* to non-T cells obtained from the mouse model, i.e. downregulation in TCR ζ chain expression was triggered, and eventually led to impaired T cell function (Figures 8 and 9).

Although only a small fraction of the T cells in the spleen directly recognizes and responds to the specific antigens that the mice were exposed to, all T cells were affected and downregulation of ζ chain expression was observed in the entire T cell population (as demonstrated in Example 1). It is thus likely that a non-antigen specific factor(s) was responsible for ζ chain downregulation which was observed in all splenic T cells.

Interestingly, Example 9 shows that exposing mice to ligands for different TLRs [4, 2, 9 and 3 (data not shown)] induced the same phenomenon of ζ chain downregulation and impaired T cell function. These ligands stimulate various cells of the innate and adaptive immune systems, and cause a general massive stimulation of the production of pro-inflammatory cytokines such as IFNγ, TNFα and inflammatory factors, which contribute to the generation of the immunosuppressive environment responsible for the induction of ζ chain downregulation and impaired T cell function. Thus, both antigen-dependent (via the TCR) and antigen-independent (via TLRs) stimulations are responsible for the creation of the immunosuppressive environment.

Example 7 shows how the TCR ζ chain downregulation induced in the model system of the invention is IFN-γ-dependent. IFNγ is secreted by myeloid cells, NK cells and activated lymphocytes. Following antigen stimulation and triggering via the various TLRs, either separately or in combination, IFNγ is secreted and most likely induces the generation/recruitment of suppressor myeloid cells that eventually will generate the immunosuppressive environment. The results indicated that IFN-γ, the paramount $T_H1$ cytokine, is very likely involved in induction of ζ hain downregulation. IFN-γ deficient mice failed to downregulate ζ chain expression following their exposure to *P. gingivalis*, despite developing, at least a partial, $T_H1$ immune response and normal $T_H2$ response against *P. gingivalis* [Houri-Haddad, Y. et al. (2002) J Dent Res 81, 395-8]. In addition, elevated concentrations of IFN-γ were measured in the chamber exudates as well as in splenic T cells from the *P. gingivalis*-treated mice. Recruitment of IFN-γ secreting cells into the spleen may be the initial step in the creation of the regulatory environment that controls ζ chain expression and T cell function under the chronic exposure to *P. gingivalis.* Thus, the present disclosure also provides a model system for the study of IFNγ function in the immune response.

The *in vivo* model system herein described mimics the suppressive conditions generated in various pathologies such as cancer, autoimmune and infectious diseases [Alberola-Ila, J. *et al.* (1997) *id ibid.*; Finke, J. H. *et al.* (1993) *id ibid.*; Nakagomi, H. *et al.* (1993) *id ibid.*; Matsuda, M. *et al.* (1995) *id ibid.*; Lai, P. et al. (1996) *id ibid.;* Kono, K.. *et al.* (1996) *id ibid.;* Kurt, R. A. *et al.*(1998) *id ibid.;* Kuss, I. *et al.* (1999) *id ibid.*; Healy, C. G. *et al.* (1998) *id ibid.*; Trimble, L. A. & Lieberman, J. (1998) *id ibid.;* Zea, A. H. *et al.* (1998) *id ibid.*; Maurice, M. M. *et al.* (1997) *id ibid.;* Liossis, S. N. *et al.* (1998) *id ibid.*], as well as chronic inflammation in general. These conditions lead to ζ downregulation and impaired T cell function. The present system showed that ζ chain downregulation is induced by a combination of sustained exposure to antigen and the concomitant development of inflammation, which are common features of these pathologies.

Hence, the model system described herein mimics the immunosuppression caused by chronic inflammation, cancer, autoimmune disorders and infectious diseases.

The inventors have demonstrated that by simply treating healthy mice with a pathogen, an antigen, or an antigen/ligands-adjuvant combination that induced an immunosuppressive environment, a dramatic decrease of ζ protein expression in all splenic T cells could be observed. Of the TCR subunits, only the ζ chain expression was affected. Interestingly, the inventors also showed that ζ chain downregulation is primarily caused by enhanced lysosomal degradation and not by the massive translocation of ζ chain to the cytoskeletal compartment, as previously suggested [Correa, M. R. et al. (1997) J Immunol 158, 5292-6] and neither by downregulation at the mRNA level. Although very low ζ chain expression was detected in T cells isolated from spleen and peripheral blood of antigen-treated mice, the cells expressed normal amounts of surface α/β-CD3, comparable to the amounts observed in T cells isolated from cancer [Alberola-Ila *et al.* (1997) *id ibid.*], rheumatoid arthritis [Maurice *et al.* (1997) *id ibid.]* and HIV [Trimble *et al.* (1998) *id ibid.]* patients. These observations are in direct contrast to the long held consensus that TCR cell surface expression is dependent on ζ protein expression [Sussman, J. J. et al. (1988) Cell 52, 85-95]. One possible explanation for this discrepancy is that the ζ chain may be substituted by the FcεRγ chain, as observed in T cells isolated from tumor-bearing mice [Alberola-Ila *et al.* (1997) *id ibid.*] and patients with lupus [Enyedy, E. J. et al. (2001) Arthritis Rheum 44, 1114-21]. However, not in all cases the FcεRγ chain substitute for the ζ chain [Maurice *et al.* (1997) *id ibid.*], including in the present model (data not shown). Alternatively, either the minute amount of ζ chain in the affected cells might be sufficient to deliver the TCR to the cell surface or another molecule may substitute for the ζ chain.

In a further aspect, the present disclosure provides a method for generating the model system herein described, comprising the following steps:

(a) exposing healthy animals to an antigen, said antigen being preferably in admixture with an adjuvant;

(b) inducing chronic systemic inflammation;

(c) evaluating the expression of TCR chains in the T lymphocytes of the antigen-exposed animals; and

(d) evaluating T lymphocyte function (*in vivo* or *ex vivo*) of the antigen-exposed animals;

wherein, the expression of TCR ζ is downregulated and the expression of TCR a, β, CD3γ, δ and ε is unchanged compared to a non-exposed animal. A non-exposed animal is an animal that has not been exposed to said antigen. These results will indicate that an immunosuppressive environment has been generated. Preferably, said T lymphocytes are obtained from spleen and/or peripheral blood.

Exposing the model animal to the test substance comprises administering said substance by any route, for example through pre-implanted chambers, or via sub-cutaneous, intravenous, intrafootpad, intraperitoneal, or in tail base injections.

Most interestingly, as demonstrated in the following Examples, the same phenomenon was observed upon exposure of the mice to different antigens, like gram-positive and gram-negative bacterial antigens, mycobacterium antigens, CpG oligonucleotides, preferably 20 to 25 base pairs, most preferably 22 base-pairs long, CpG oligonucleotides in complex with an antigen, lipopolysacharide (LPS) of different sources and zymosan. In addition, the same phenomenon was induced upon subjecting mice to poly-IC, an activator of TLR3 (data not shown). Preferably, the antigen to be used for injecting the animal model of the invention is in admixture with an adjuvant. Usually, incomplete Freund's adjuvant and liposomes are used. The inventors have also observed that the injection of complete Freund's adjuvant, without any additional antigen, can induce the downregulation of TCR ζ chain in the T cells of the injected animals, likely due to the inactive tuberculosis mycobacterium antigen present in said reagent.

All the results presented herein suggest that ζ chain downregulation is a phenomenon that occurs after repeated exposure to practically any antigen that can generate an inflammatory response, and thus it is not a phenomenon specifically triggered by *P. gingivalis* antigens.

Moreover, the same results were obtained after delivering the antigen to the animals by several means, like pre-implanted chambers, or via subcutaneous, intrafootpad, or intraperitoneal injections.

In an even further aspect, the present disclosure refers to an in *vivo* screening method for substances that upregulate and/or prevent the downregulation of the TCR ζ chain *in vivo*, comprising the following steps:

(a) exposing the model animal system of the disclosure to a test substance;

(b) evaluating the expression of TCR chains in the T lymphocytes of the test substance-exposed animal;

(c) evaluating T cell function;

wherein, if the expression of TCR ζ is higher than the expression of TCR ζ in a model system which has not been exposed to said test substance, said test substance is an inhibitor of the downregulation of TCR ζ. Preferably, said T lymphocytes are obtained from the spleen and/or peripheral blood of the model animal.

Similarly, the disclosure also enables two *ex* vivo screening methods for substances that can upregulate or prevent the downregulation of the TCR ζ chain *in vitro.*

The first *ex vivo* screening method comprises the following steps:

(a) obtaining a body fluid and/or tissue sample from an animal model system for TCR ζ downregulation, as defined in the disclosure;

(b) separating non-T cells from said sample;

(c) providing T cells from a healthy animal;

(d) establishing a mixed cell culture with said non-T and T cells, wherein, after incubating these two cell populations together, TCR ζ expression is downregulated in said T cells;

(e) exposing said cell culture to a test substance for an effective time period;

(f) evaluating the expression of TCR chains in the T cells in the culture;

wherein if the expression of TCR ζ in said T cells is higher than in the T cells of a non-exposed corresponding mixed culture, said test substance is an inhibitor of the downregulation of TCR ζ expression.

In the second *ex vivo* screening method, both T and non-T cells are derived from a model system animal (i.e., both cell populations were exposed to the antigen *in vivo*). Accordingly, said second *ex vivo* screening method comprises the following steps:

(a) obtaining a body fluid and/or a tissue sample from an animal model system for TCR ζ downregulation, as defined in the disclosure;

(b) extracting T and non-T cells from said sample;

(c) establishing a mixed cell culture with said non-T and T cells, wherein, after incubating these two cell populations together, TCR ζ expression is downregulated in said T cells;

(d) exposing said cell culture to a test substance for an effective time period;

(e) evaluating the expression of TCR chains in the T lymphocytes in the culture;

wherein if the expression of TCR ζ in said T cells is higher than in the T cells of a non-exposed corresponding mixed culture, said test substance is an inhibitor of the downregulation of TCR ζ expression.

Upon extracting the cells (T and non-T) from said sample, said cells may be put in cell culture as is or, alternatively, the T and non-T cells may be separated, and then put in culture. If, and when, T and non-T cells are separated, the ratios of these cells may be manipulated before establishing the mixed culture of step (c) of the above method.

Most preferably, the T cells are obtained from spleen and/or peripheral blood.

In addition, it is important to mention that the non-T cells employed in the two screening methods described herein are myeloid cells, either total myeloid cells or specific myeloid subpopulations.

Based on the results present in Example 8, a mixture between T and non-T cells from untreated animals may be used as a control wherein TCR ζ downregulation was never induced in the first place. On the other hand, in a mixture between T cells obtained from the model system animals and non-T cells obtained from healthy animals, the TCR ζ expression is recovered in the T cells, and this T cell population could serve as a term for comparison for the level of expression of ζ chain that should be achieved in the T cells treated by the substance being tested.

A candidate substance suitable for testing in the screening methods of the disclosure may be selected from the group consisting of protein based, carbohydrates based, lipid based, natural organic based, synthetically derived organic based, inorganic based, and peptidomimetics based substances.

Hence, the present disclosure provides the use of the model system of the disclosure for screening of substances that upregulate or prevent the downregulation of the TCR ζ chain. A desirable test substance will be one that can elevate or increase the levels of TCR ζ chain expression in T cells obtained from the animal model system as defined in the invention, in particular in comparison to the levels of TCR ζ chain expression in cells of the same origin, but which have not been treated with said test substance.

Preferably, such substance may be a product of any one of positional scanning of combinatorial libraries of peptides, libraries of cyclic peptidomimetics, and random or dedicated phage display libraries.

Thus, in one additional aspect the present disclosure provides a substance that inhibits the downregulation of TCR ζ chain expression, wherein said substance is identified by one of the above-described screening methods.

One mechanism to explain the phenomena observed in the model system of the disclosure would be that non-T cells in the spleen such as macrophages and granulocytes could be activated by IFN-γ to secrete factors that induce chain downregulation in splenic T cells. Indeed, macrophages and/or granulocytes accumulate in the spleen of tumor-bearing hosts [Otsuji , M. et al. (1996) Proc Natl Acad Sci USA 93, 13119-24; Almand, B. et al. (2001) J Immunol 166, 678-89], as well as in hosts undergoing other inflammatory processes, including those of the model presented herein. Reactive oxygen metabolites secreted by macrophages and/or immature myeloid cells isolated from cancer-bearing hosts induce ζ chain downregulation when tested in *vitro* [Otsuji , M. *et al.* (1996) *id ibid.*; Kono , K. et al. (1996) Eur J Immunol 26, 1308-13; Schmielau, J. et al. (2001) Cancer Res 61, 4756-60]. Moreover, a recent study showed that oxygen metabolites secreted by immature myeloid precursors derived from tumor-bearing mice can inhibit in *vitro* proliferation of naive T cells in an IFNγ-dependent manner [Kusmartsev, S. A. et al. (2000) Immunol 165, 779-85].

In yet another aspect, the present disclosure provides the use of the model system of the disclosure for screening of cells to be used in immunotherapeutic regimens. Such screening shall be very useful in determining the timing and the mode of the applied immunotherapy. According to the sensitivity of the applied cells (variety of T lymphocytes or NK cells) to the harmful environment, the therapeutic regiments could be improved and/or the timing of the applied therapy could be modified according to the degree to which the environment is harmful. By means of the present invention, the environment - of the subject in need of immunotherapy - could be tested as to whether it is harmful or not and when it appears, through measuring TCR ζ expression level. Thus, therapy could be directed to the time in which the environment is not spoiled yet. Therefore, testing TCR ζ expression levels may serve as a prognostic tool for the presence of an immunosuppressive environment. Alternatively, said model system could be used for the screening of vaccination therapies, wherein functional immune system is required.

The immunosuppressive environment is composed of cells, mainly myeloid cells, secreted factors and cytokines. The immunosuppressive environment is generated following sustained antigen-dependent (via the TCR) and/or antigen independent (via TLRs) stimulation. This stimulation causes IFN-γ secretion, inducing the generation/recruitment of suppressor myeloid cells, secreting factors and cytokines, and the combination of these (cells+factors) makes up the immunosuppressive environment.

Active immunotherapy is an approach to the treatment of cancer based on the presence of tumor-associated antigens. In certain tumors, proteins are preferentially expressed on the surface of the tumor cells, and thus can be potentially used as targets in T-cell mediated cancer immunotherapy. Immune recognition of these antigens occurs usually via specific CD8+ CTL (cytotoxic T lymphocytes), although MHC Class II-binding epitopes recognized by CD4+ T cells have also been described. Under optimal circumstances, initiation of an immune response is triggered by peptide presented by the MHC complexes expressed by host antigen-presenting cells (APC), and additionally requires multiple cofactors provided by APC. After initial activation, CTL induced by APC interactions are thought to migrate throughout the host, recognize the same MHC/peptide complex in the tumor cells, and be triggered to kill them. This antigen-specific cytolysis is mediated largely via induction of apoptosis.

There are also passive immunotherapeutic strategies using patient's T cells stimulated *in vitro* against tumor cells, or T cells engineered to express antibody directed against tumor antigens that will direct the cells specifically to the tumor.

The immunotherapy approach for treating cancer is based on the premise that the immune system of the subject suffering from the cancer is functional. Based on this premise, there are two assumptions: (1) that the immune system will be able to develop an active immune response; and (2) if the immunotherapy involves the administration of immune cells, that these will not be affected by the environment of the host and will be able to perform their function. As mentioned in the Background, such situation is not always the case. Often cancer patients have developed a state of immunosuppression, which, as demonstrated by the present inventors, is evidenced by the downregulation of TCR ζ expression. Therefore, being able to evaluate the ability of the patient to respond to such immunotherapeutic approaches is crucial for the success of the same. The present invention provides a tool, or a prognostic marker, for evaluating the presence of an immunosuppressive or immunosuppressed environment.

In view of the above, the present invention provides the use of TCR ζ chain protein level as a marker for an immunosuppressive environment, wherein downregulation of TCR ζ chain expression means the presence of an immunosuppressive environment, as defined in the claims. Preferably, said immunosuppressive environment is a result of any one of the following conditions: chronic inflammation, cancer, infections and autoimmune disorders.

Similarly, the present invention also provides the use of TCR ζ chain protein level as a prognostic marker for the emergence of an immunosuppressive environment, wherein inhibition of TCR ζ chain means the development of an immunosuppressive environment, as defined in the claims.

The use of TCR ζ chain expression as a prognostic marker is especially important for monitoring pre-immunotherapy patients.

Although surface α/β-CD3 expression on T cells isolated from *P. gingivalis*-treated mice was normal, the receptor was functionally impaired both *in vitro* and *in vivo*. The latter is reflected by the reduced ability of *P. gingivalis*-treated mice to clear an influenza virus infection that started after the last *P. gingivalis* injection (Example 4). Once the exposure of the mice to *P. gingivalis* is stopped, ζ chain levels and T cell function gradually return to normal (data not shown). Therefore, the diminished ability of the treated mice to contend with a viral infection reflects the immune system as it was already recovering, and may underestimate the full extent of *P. gingivalis* mediated suppression of the immune response. It is important to note that the impaired immune response was directed against an antigen (influenza virus) that differs greatly from those to which the mice had been previously exposed (the *P. gingivalis* antigens), indicating that the *P. gingivalis* treatment induced a generalized T cell dysfunction. Indeed, the *P. gingivalis* treatment induced ζ downregulation in both CD4+ and CD8+ T cells. Similarly, following the *P. gingivalis* treatment, all T cells highly expressed surface CD44 and elevated levels of tyrosine-phosphorylated proteins were observed (data not shown).

The inventors propose that ζ chain downregulation could be a normal mechanism to control an excessive and potentially hazardous immune response. In the case of a time-limited exposure to antigen, or in acute bacterial or viral infections, the transient ζ chain downregulation and T cell unresponsiveness that ensues may help restore the balance of a "superactivated" immune system. Indeed, in the present model the mice regained normal ζ chain expression and T cell function within 10 days following the last antigen injection (data not shown). In contrast, in pathological conditions exhibiting ζ downregulation, the continuous presence of pathogen or tumor antigens and chronic inflammation most likely prevents such a recovery, thus contributing to the pathological phenotype of the diseases.

In a developing inflammatory environment, the accumulation of regulatory CD11b+GR1+ myeloid suppressor cells (MSCs) is observed. This population is heterogeneous, and it has been detected in lymphoid organs during tumor growth, in graft-versus-host reactions and in infectious diseases. In all cases, impaired T-cell responses to TCR-mediated stimuli are observed. MSCs have an important role in the regulation of the inflammatory process and in the control of T-cell responses. MSC recruitment can have an important role in the control of excessive immune responses. The expansion of MSC populations in the lymphoid organs of infected or immunized mice is transient. By contrast, under conditions of chronic inflammation, such as during tumor progression and chronic infection, the number of MSCs remains high and immunosuppression is maintained. MSCs suppress the activation of both CD4+ and CD8+ T cells in an antigen- and MHC-independent manner. Both cell-cell contact between MSCs and T cells, and secreted compounds, are required for the inhibitory activity of MSCs. Therefore, hindering the mechanisms used by MSCs to suppress T-cell function seems to be the most promising therapeutic strategy for restoring T-cell function.

Thus in a last aspect, the present disclosure provides a method of restoring immune function in a subject suffering from immunosuppression, said method comprising inhibiting the activity of CD11b+Gr1+ myeloid suppressor cells.

The present invention is defined by the claims, the contents of which are to be read as included within the disclosure of the specification, and will now be described by way of example with reference to the accompanying Figures.

It must be noted that, as used in this specification and the appended claims, the singular forms "a", "an" and "the" include plural referents unless the content clearly dictates otherwise.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

## Examples

### Experimental Procedures

#### Animals

Female BALB/c, C57B1/6 or C3H mice, aged 6 to 7 weeks, were bred at the Hebrew University SPF facility. IFN-γ deficient mice (IFNγ-/-) on a BALB/c background were obtained from The Jackson Laboratory (Bar Harbor, Maine). Animal usage was carried out according to protocols approved by the Hebrew University-Hadassah Medical School Institutional Animal Care and Use Committee.

#### The model system

The inventors designed an in vivo experimental system based on sustained exposure of mice to various antigen-adjuvant combinations. The exposure to the antigen was performed using different regimens: 1) a modification of the mouse subcutaneous chamber model used for analysis of host-parasite interactions [Genco, C. A. & Arko, R. J. (1994) id ibid; Houri-Haddad, Y. et al. (2000) id ibid.], and 2) subcutaneous, intrafootpad and intraperitoneal injections of antigen/ligands-adjuvant mixtures.

Briefly, in the chamber system, normal (healthy) mice were implanted subcutaneously with chambers constructed from coils of titanium wire via a mid-dorsal incision. After 1 week (Fig. 1a), mice were subcutaneously injected with 0.1 ml of $10^{10}$ CFU/ml heat-killed Gram-negative bacteria, P.g. [Baker, P. J. et al. (1994) Arch Oral Biol 39, 1035-40; Kesavalu, L. et al. (1992) Infect Immun 60, 1455-64; Houri-Haddad et al. (2000) id ibid.] mixed with 0.1 ml of incomplete Freund's Adjuvant (IFA) (Sigma, St. Louis). Following 1 and 2 antigen injections, the mice received an intra-chamber injection of heat-killed P.g. (0.1 ml of $10^{10}$ CFU/ml) in PBS (Fig. 1a). Unless stated otherwise, splenocytes or peripheral blood cells were harvested one day after the last injection (day +1). Control mice were subjected to the same protocol, but injected with PBS instead of the bacterial antigen. For experiments using chicken ovalbumin (OVA) (Sigma, St. Louis) as antigen, the same protocol was maintained except that the first injection was with 100μg OVA adsorbed to Al(OH)$_3$ (Alum) (Sigma, St. Louis), while the second and third injections used 100μg OVA in PBS. Experimental groups included at least six animals, and each experiment was repeated at least three times. In the subcutaneous, intrafootpad and intraperitoneal injections, the antigens were injected in combination with liposomes. The antigens used were: heat-killed mycobacteria, TLR-2 ligands like LPS from *P. gingivalis* and zymosan, TLR-9 ligands like CpG ODN (SEQ. ID. NO.1: 5'-TGACTGTGAACGTTCGAGATGA-3'), its mutant (which was used as a control) (SEQ. ID. NO.2: 5'-TGACTGTGAAGGTTGGAGATGA-3'), TLR-4 ligands like LPS from *E. coli*, and TLR-3 ligands like poly-IC (data not shown).

The adjuvants used were Complete Freund's Adjuvant (CFA), Incomplete Freund's Adjuvant (IFA), and liposomes.

CFA (Sigma): Each ml of F5881 contains 1 mg of heat-killed and dried *Mycobacterium tuberculosis* (strain H37Ra, ATCC 25177), 0.85 ml paraffin oil and 0.15 ml of mannide monooleate. 100□l of emulsion 1:1 CFA:OVA was injected subcutaneously 3 times with one week intervals.

IFA (Sigma): Each ml of F5881 contains 1 mg of heat-killed and dried *Mycobacterium tuberculosis* (strain H37Ra, ATCC 25177), 0.85 ml paraffin oil and 0.15 ml of mannide monooleate. 100$\mu$l of emulsion 1:1 IFA:OVA was injected subcutaneously 3 times with one week intervals.

Liposomes: Large multilamellar vesicles (MLV, mean diameter 1.3-1.5 □m) consisting of dimyristoyl-phosphatidylcholine (DMPC) and dimyristoyl-phosphatidylglycerol (DMPG) (both from Lipoid GmbH, Ludwigshafen, Germany), at a DMPC:DMPG mole ratio of 9:1, using the following procedure. Lipids (10-50 mg) were dissolved in 1 ml of tertiary butanol and then sterilized by filtration (GF92, Glasforser, Vorfilter no. 421051, Schleicher & Schuell, Dassel, Germany). The sterile lipid solution was frozen at -70°C and then lyophilized for 24 hours to complete dryness. The dried lipids could be stored at 4°C for more than 1 year without significant (<10%) lipid degradation or loss of encapsulation capability. Upon need, the lipid powder was hydrated with the antigen (OVA) and/or TLR ligand solution (in saline).

Alternatively, heat-killed mycobacteria, gram-positive bacteria, heat-killed influenza virus or various Toll-like receptor ligands were injected (3-4 times) intrafootpad and intraperitoneal in combination with adjuvants, to obtain the same results as above.

Cell surface biotinylation, lysis and immunoblotting

The analyses were performed either on the splenocyte population or on T cells isolated from the spleen using Fluorescein isothyocyanate (FITC) labeled anti-Thy1.2 (Pharmingen, San Diego, California) and anti-FITC conjugated magnetic beads (Miltenyi Biotec, Gladbach, Germany). Cells (200 x $10^6$/ml) were lysed with Tris-NaCl buffer containing 0.5% Triton X-100, as previously described [Caplan & Baniyash (1996) *id ibid.*]. Following 30 minutes of lysis on ice, the proteins were resolved using one-dimensional reducing or two-dimensional non-reducing/reducing 13% SDS-PAGE, as previously described [Franco, J. L. et al. (1995) Cancer Res 55, 3840-6]. Western blot analysis was performed using specific antibodies: monoclonal anti-$\zeta$ (H146) [Rozdzial, M. M. et al. (1994) J Immunol 153, 1563-80], polyclonal anti-CD3$\gamma\delta$ [Samelson, L.E. et al. (1986) J Immunol 137, 3254-8] and anti-$\epsilon$ (Santa Cruz Biotechnology). The specific antibodies were detected by incubation with protein A (Amersham, Piscataway, New Jersey) or anti-goat antibodies conjugated to horseradish peroxidase (HRP) (Jackson, West Grove, Pennsylvania), followed by an enhanced chemiluminescence (ECL) and exposure onto Kodak X-ray films. Cell surface biotinylation was performed as previously described [Caplan, S. & Baniyash, M. (1996) J Biol Chem 271, 20705-12] and biotinylated proteins were visualized by streptavidin-HRP (Jackson, West Grove, Pennsylvania) followed by ECL.

Immunostaining, FACS and confocal analysis.

The antibodies used for cell surface labeling were FITC-labeled anti-Thy 1.2 and anti-CD8, Phycoerythrin-labeled anti-CD4 and anti-CD45R/B220, and biotinylated anti-CD3s and anti-TCR$\alpha$/$\beta$ that were detected by streptavidin-Cy5. All the antibodies and second reagents were purchased from Pharmingen, San Diego, California. Cells were precoated with anti-mouse CD16/CD32 and then incubated for 30 minutes at 4° C with the specific labeled antibodies. Following washes, the relevant samples were incubated with a second step reagent. For intracellular staining of the $\zeta$ and CD3$\epsilon$ chains, the cells were first stained for cell surface T cell marker, washed, fixed for 4 minutes with 1% paraformaldehyde at room temperature and permeabilized for 4 minutes with 0.2% Triton X-100 and 0.2% BSA (Sigma, St Louis, MO). Cells were washed, incubated for 10 minutes with hamster serum, and then with biotin-labeled anti-CD3$\epsilon$ (Pharmingen, San Diego, California) or with biotinylated monoclonal anti-$\zeta$ (H146) [Harlow, E. & Lane, D. (1999) Antibodies: a laboratory manual, Cold Spring Harbor Laboratory Press, Cold Spring Harbor, New York] that were detected using streptavidin-Cy5 (Jackson, West Grove, PA). Samples were analyzed in a FACSCalibur using Cell Quest software (Becton Dickson, Mountain View, CA) or under a confocal microscope (LSM 410, Zeiss, Jena, Germany).

Analysis of lymphocyte function

For mitogen-induced cell proliferation [Leshem, B. et al. (1999) J Immunol Methods 223, 249-54], splenocytes (2 x $10^6$ cells/ml) were cultured in the presence of anti-CD3$\epsilon$ (145-2C11), 2.5 $\mu$g/ml concanavalin A (Con A, Biomakor, Israel), 2 ng/ml phorbol 12-myristate13-acetate (PMA, Sigma) combined with 0.2 $\mu$M calcium ionophore (Sigma), or in medium alone. Following 48 h of incubation at 37° C, 1 µCi of [³H]-thymidine was added to each well. Cells were harvested 8 h later using a Tomtec (USA) cell harvester and counted in a MicroBeta scintillation counter (Wallac, Finland). The mixed leukocyte reaction (MLR) was performed as previously described [Leshem, B. *et al.* (1999) *id ibid.*] and cell proliferation was evaluated according to the levels of the cell [³H]-thymidine uptake. Cell-mediated cytotoxicity assays were performed as previously described [Leshem, B. & Brass, D. (1998) Immunology 95, 409-18]. Cytotoxic activity, measured by ⁵¹Cr release, is presented as percentage specific cytotoxicity:

$$100 \times \frac{(\text{experimental cpm} - \text{background cpm})}{(\text{maximal cpm} - \text{background cpm})}$$

BrdU Staining and T cell surface labeling

Splenocytes were activated with anti-CD3ε antibodies, ConA or PMA+ionophore as described above. During the last 3 hours of activation, 15µM of BrdU (Sigma, St. Louis) were added. Cells were harvested, pre-coated with anti-mouse CD16/CD32 and stained with biotinylated anti-Thy1.2 followed by incubation with Cy5-conjugated Streptavidin. Cells were washed, diluted in cold 0.15 M NaCl and fixed with cold 95% ethanol. After 30 min on ice, cells were washed with PBS and incubated for 30 min at room temperature in PBS / 1% paraformaldehyde / 0.01% Tween-20. Cells were centrifuged, incubated for 10 min at room temperature in DNase I (Sigma, St. Louis) solution [50 U of DNase I per ml in 4.2 mM MgCl₂, 0.15 M NaCl, pH 5] and washed with PBS. The cells were then incubated with FITC-conjugated anti-BrdU (Dako, Denmark) for 30 min at room temperature, washed and analyzed by flow cytometry. Non-pulsed activated cells were used as a control for the anti-BrdU staining.

CFSE staining

Splenocytes were suspended in PBS without Ca²⁺, Mg²⁺, with CFSE (Molecular Probes) added to a final concentration of 0.5-5 µM. The suspension was then incubated at 25°C for 8 minutes. At the end of this incubation, fetal calf serum (FCS) at the same volume was added and the cells washed twice in RPMI with 8% FCS serum. CFSE-labeled splenocytes were activated with anti-CD3ε or PMA plus ionophore as described before. CFSE is retrained, but dispersed equally into subsequent generations, so each division cohort produces one half the fluorescence of its parent cohort in flow cytometry. Thus, following staining of the T cells with anti-Thy1.2 (as above), the number of T cell divisions could be detected.

Influenza virus infection and virus titer determination in the lung

Mice were anesthetized and intranasally infected with an influenza virus (A/PR/8/34) at 10²·⁵ 'egg infectious dose 50%' (EID₅₀) units one day after the last *P. gingivalis* injection (see Fig. 4a). At the indicated time intervals, the lungs were removed and homogenized. The amount of viable virus in the lungs was detected as previously described [Reed, J. L. & Muench, M. (1938) Am. J. hyg. 27, 493-497]. Virus titer was calculated using the method of Reed and Muench [Reed, J. L. & Muench, (1938) *id ibid.*] and is presented in log₁₀ units.

Measurement of specific serum IgG1 and IgG2a antibodies and IFN-γ and IL-10 in chamber exudates

Serum samples were collected at day +9 of the experiment and analyzed by ELISA for the levels of *P.g.*- or OVA-specific IgG1 and IgG2a antibodies as previously described [Houri-Haddad *et al.* (2000) *id ibid.*]. IFN-γ and IL-10 concentrations in the chamber exudates were determined at 4 and 24 h, respectively after the third *P.g.* injection by two-site ELISA [Frolov, I. et al. (1998) Immunology 93, 923-8].

RNA preparation and Northern blot analysis

T cells were isolated from the spleen of control and *P. gingivalis*-treated mice as mentioned above and total RNA was prepared using the EZ-RNA kit (Biological Industries, Israel). 20µg of each sample were subjected to Northern blot analysis, as previously described [Bronstein-Sitton et al. (1999) J Biol Chem 274, 23659-65.] ζ mRNA levels were detected following hybridization with P³²-labeled ζ-specific cDNA probe. The amount of applied RNA in each group was analyzed following the hybridization with control P³²-labeled ribosomal-specific cDNA probe L32.

**Example 1 (Reference example)**

**Repetitive *P. gingivalis* exposure induces $\zeta$ downregulation,**

To identify the immunological milieu that induces $\zeta$ downregulation, an in vivo experimental system was established, in which healthy mice were "chronically" exposed to heat-killed *P. gingivalis.* The mice were injected once subcutaneously with heat-killed *P. gingivalis* in incomplete Freund's Adjuvant (IFA) and 1 and 2 weeks later, the mice received intra-chamber injections with heat-killed *P. gingivalis* in PBS. One day following the third injection, the levels of $\zeta$ expression were examined in the spleen. Western blot analysis of cell lysates from the whole spleen population or isolated T cells revealed that the *P. gingivalis* treatment induced dramatic $\zeta$ downregulation (Fig. 1b). Similar results were obtained with three other antibodies, each recognizing a different epitope on the $\zeta$ molecule (data not shown). The downregulation was specific for the $\zeta$ chain as CD3 $\epsilon$ (Fig. 1b, lower panel) and the other TCR subunits (data not shown) were unaffected. To rule out the possibility that loss of $\zeta$ chain is due to proteolytic degradation by enzymes released from granulocytes or macrophages during cell lysis [Franco, J. L. *et al.* (1995), *id ibid.*], $\zeta$ protein expression was examined in intact splenocytes by immunofluorescence. Figure 1C shows a dramatic loss of $\zeta$ protein. Remarkably, reduction of $\zeta$ expression was induced in all T cell populations ($CD4^+$ and $CD8^+$ cells) in the spleen of the treated mice, as shown by FACS analysis (Fig. 1d, upper panel). This is in contrast to CD3 $\epsilon$ and the TCR $\alpha/\beta$ subunits whose expressions were not affected by the *P. gingivalis* treatment (Fig. 1d, middle panel). Despite the loss of $\zeta$ chain, T cells isolated from spleens of treated mice expressed normal levels of cell surface CD3 and TCR $\alpha/\beta$ subunits (Fig. 1d, lower panel). Moreover, the cell surface expressed TCR complexes were correctly assembled and contained all the subunits (Fig. 1e). The relative T and B cell distribution was maintained in the spleen of P. gingivalis-treated mice (Fig. 1f). Although an increase of non-T and non-B cell population was detected in the spleens of P. gingivalis-treated mice (Fig. 1f), the total number of T cells in the spleen of treated and control groups was similar. This is due to the enlargement of the spleen in treated ($\sim 200 \times 10^6$ splenocytes/spleen) as compared to control mice ($\sim 100 \times 10^6$ splenocytes/spleen). Thus, repetitive exposure of normal mice to *P. gingivalis* induced $\zeta$ downregulation. These experiments were done also with peripheral blood lymphocytes (obtained from peripheral blood), and similar results were obtained (data not shown).

**Example 2**

**$\zeta$ downregulation is due to lysosomal degradation**

The dramatic loss of $\zeta$ expression observed in splenocytes isolated from *P. gingivalis*-treated mice could be regulated at various levels. One possibility is that the $\zeta$ chain translocates from the detergent-soluble fraction (analyzed in Fig. 1b) to the detergent-insoluble (cytoskeletal) fraction, as demonstrated previously in activated T cells [Caplan, S. & Baniyash, M. (1996) *id ibid.*; Caplan, S. et al. (1995) Proc Natl Acad Sci USA 92, 4768-72]. The present analyses revealed that reduced $\zeta$ expression was also detected in the cytoskeletal fraction (data not shown). Further, the inventors examined whether $\zeta$ expression in the *P. gingivalis* model was regulated by a lysosomal-dependent degradation process, as was previously shown in both resting and activated T cells [Bronstein-Sitton, N. *et al.* (1999) *id ibid.*; Valitutti, S. *et al. (1997) id ibid.*; D'Oro, U. et al. (1997) Immunity 7, 619-28]. To this end, splenocytes isolated from P. *gingivalis*-treated mice were treated with the lysosome inhibitors $NH_4Cl$ or bafilomycin A. Incubation with both inhibitors largely restored $\zeta$ protein expression (Fig. 2a). Incubation of splenocytes derived from the treated mice with proteosome inhibitors had no effect on $\zeta$ expression (data not shown). $\zeta$ mRNA levels in splenocytes isolated from control and *P. gingivalis*-treated mice were also tested and found to be similar in both groups (Fig. 2b). These results indicate that the TCR $\zeta$ downregulation observed in the present *in vivo* model system is controlled at the post-translational level, mainly by enhanced lysosomal degradation.

**Example 3**

**Impaired T cell function in *P. gingivalis*-treated mice**

Next the inventors determined whether $\zeta$ chain downregulation induced by the *P. gingivalis* treatment also correlated with diminished immune function, as has been observed in a variety of tumors, several infectious diseases, and in autoimmune disorders. To this end, the immune responsiveness of T cells from P. gingivalis-treated mice was examined. Proliferation of splenocytes derived from *P. gingivalis*-treated mice in response to anti-CD3 or Con A, as measured by the uptake of $[H^3]$-thymidine, was significantly decreased compared to control splenocytes (Fig. 3a). In contrast, proliferative responses of control and *P. gingivalis*-derived cells stimulated with a combination of PMA and $Ca^{2+}$ ionophore were similar. These results indicate that while the immediate TCR-mediated signaling events were impaired in T cells from *P. gingivalis*-treated mice, the downstream signaling machinery in these cells was intact. Using FITC-labeled anti-BrdU antibodies, together with Cy5-labeled anti-Thy1.2 antibodies for T cell labeling, the degree of DNA synthesis of individual T cells was measured. T cells in the spleen of P. gingivalis-treated mice also exhibited diminished proliferation in response to TCR-mediated stimuli as compared to controls (Fig. 3b). Impaired proliferative responses were also evident when cells isolated from *P. gingivalis*-treated mice were tested in a MLR reaction (Fig. 3c). The potential effector function of the cells isolated from the treated mice was also examined. Cells isolated from *P. gingivalis*-treated mice were unable to mount a normal cytotoxic reaction in response to allogeneic target cells (Fig. 3d). Thus, T cells in the spleen of *P. gingivalis*-treated mice exhibit impaired *in vitro* function that correlate with low levels of TCR $\zeta$ chain expression.

## Example 4

### *P. gingivalis*-treated mice exhibit reduced immunity against viral infection

To determine whether the impaired function of T cells expressing low levels of $\zeta$ chain observed *in vitro* was also reflected *in vivo*, the response of *P. gingivalis*-treated mice to infection with a sub-lethal dose of influenza virus was examined. Influenza infection in mice is well characterized, with virus clearance depending on T cells [Wells M. A. et al. (1981) J Immunol 126, 1042-6]. Mice were infected with influenza virus one day after the last *P. gingivalis* or PBS injection. Mice were sacrificed at various time points (Fig. 4a) and the virus titers in the lung measured. Control mice exhibited the expected kinetics of viral clearance [Ada, G. L. & Jones, P. D. (1986) Top Microbiol Immunol 128, 1-54; Gerhard, W. et al. (1997) Immunol Rev 159, 95-103] and by day 9 post infection had successfully eliminated the virus. In contrast, *P. gingivalis*-treated mice were unable to clear the virus under the same conditions. These results showed that the impaired T cell function observed *in vitro*, associated with $\zeta$ downregulation, is also observed *in vivo.*

## Example 5

### Sustained *P. gingivalis* exposure induces $\zeta$ downregulation

Having established that treating normal mice with *P. gingivalis* could induce $\zeta$ chain downregulation and impaired T cell function, the inventors examined whether repeated exposure to antigen was necessary for the induction of $\zeta$ downregulation, or whether one or two *P. gingivalis* injections could induce the same phenomenon, if enough time was given for a response. To this end, mice were exposed to a different number of *P. gingivalis* injections, and $\zeta$ protein expression in the splenocytes was analyzed at different time points (Fig. 5a). In mice that received only the first injection (Fig. 5b), the amount of $\zeta$ protein remained normal at day -8, and at day +1 only a slight decrease in $\zeta$ expression was observed. Mice that received two *P. gingivalis* injections showed only a slight decrease, if any, in $\zeta$ expression. Maximal $\zeta$ downregulation was only observed after the third *P. gingivalis* injection, indicating that in the model herein developed, a sustained exposure to antigen is indeed necessary for the induction of $\zeta$ downregulation.

## Example 6

### $\zeta$ chain downregulation is T$_H$1-dependent

Next the inventors determined whether the dominant T$_H$1 immune response induced by the *P. gingivalis* treatment was required for $\zeta$ chain downregulation. For this purpose, $\zeta$ protein expression and T cell function were compared in *P. gingivalis*-treated mice with mice treated with OVA in Alum, a regimen that elicits a T$_H$2 response. The latter group was injected once subcutaneously with OVA in Alum and then twice into the chamber with OVA in PBS, similar to the *P. gingivalis* treatment. The treatment with OVA induced a strong T$_H$2-dependent immune response as shown by the presence of specific OVA antibodies of the IgG$_1$ isotype and not of the IgG$_2$a isotype (Fig. 6a, right panel). In spite of the potent immune response, OVA treatment did not induce profound $\zeta$ downregulation as observed in the *P. gingivalis*-treated mice (Fig. 6b). In addition, the correlation between $\zeta$ chain expression and T cell function was maintained: T cells in the spleen of mice treated with OVA exhibited normal function, compared T cells from the spleens of *P. gingivalis*-treated mice that were functionally impaired (Fig. 6c). High concentrations of both specific IgG$_1$ and IgG$_2$a were measured in the sera of *P. gingivalis*-treated mice (Fig. 6a, left panel), consistent with the notion that, over time, an initial T$_H$1 immune response switches to a T$_H$2 response [Spellberg, B. & Edwards, J. E., Jr. (2001) Clin Infect Dis 32, 76-102]. Therefore, a mixture of T$_H$1 and T$_H$2 immune responses was detected. Thus, a T$_H$1 immune response is required for the induction of $\zeta$ downregulation, and the presence of a T$_H$2 immune response has no effect on $\zeta$ chain expression.

**Example 7**

**ζ chain downregulation is IFNγ-dependent**

The primary difference between $T_H1$ and $T_H2$ responses is the cytokine profile produced by each subset. The inventors therefore examined the cytokines generated in vivo by analyzing the chamber exudates. There was no great difference in the concentrations of the type 2 cytokine interleukin 10 (IL-10) measured in *P. gingivalis*- versus OVA-treated mice. However, there were profound differences in the amount of the type 1 cytokine, IFN-γ (Fig. 7a, right panel). Although in OVA-treated mice IFN-γ was practically undetectable, in *P. gingivalis*-treated mice, IFN-γ was present at high concentrations in the chamber. Moreover, flow cytometry analysis of multicolor staining for intracellular cytokines revealed that T cells in the spleen of *P. gingivalis*-treated mice secreted either IL-10 or IFN-γ, whereas T cells in the spleen of OVA-treated mice secreted only IL-10 (data not shown). The observed cytokine profile in the *P. gingivalis*-treated mice also showed that a mixed of $T_H1$-$T_H2$ immune response was elicited. These results further support the conclusion that a $T_H1$ immune response is required for the induction of ζ downregulation, and the presence of a $T_H2$ immune response has no effect on ζ chain expression.

The elevated IFN-γ production detected in the *P. gingivalis*-treated mice suggested that IFN-γ might play a role in ζ chain downregulation. Indeed, T cells from IFN-γ deficient mice treated with *P. gingivalis* exhibited only a slight ζ chain downregulation compared to the dramatic ζ downregulation observed in wild type-treated mice (Fig.7b). Thus, IFN-γ plays a major role in the process leading to ζ chain downregulation in all T cell populations. To examine whether IFN-γ directly or indirectly affects all T cell populations in the spleen, T cells from control mice were exposed to IFN-γ in the presence or absence of control non-T cell populations *in vitro.* These conditions did not affect ζ chain expression (Fig.7c, upper panel). In contrast, exposure of control T cells to the non-T cell population isolated from the spleen of *P. gingivalis*-treated mice induced pronounced ζ chain downregulation. Moreover, when isolated T cells from *P. gingivalis*-treated mice were incubated for 24h *in vitro* with or without IFN-γ, they recovered ζ chain expression (Fig.7c, lower panel). In contrast, when these T cells were incubated together with non-T cells from the spleen of *P. gingivalis*-treated mice, ζ downregulation was maintained for 24 hours in culture. Thus the non-T cell population from the spleen of the *P. gingivalis*-treated mice is required for the maintenance of low ζ expression levels and neither IFN-γ nor normal non-T cell populations could replace this environment. Anti-IFN-γ did not neutralize the regulatory effect of the non-T cell population isolated from the *P. gingivalis*-treated mice (data not shown). These results suggest that IFN-γ most likely indirectly affects the T cells via a non-T cell population(s) that is recruited to the spleen of the *P. gingivalis*-treated mice during the chronic inflammatory response (Fig. 1f).

**Example 8**

**Normal T cells downregulate ζ expression when injected into the *P. gingivalis*-treated mice.**

Normal PKH-labeled splenocytes were injected at day -2 into the spleen of host mice. At day -1 the mice were injected with heat-killed *P. gingivalis.* At days -1 (prior to the antigen injection) 1, 5 and 12 (following antigen injection) the mice were sacrificed, spleens were harvested and ζ expression in the hosts' (Fig. 8a) and donor (Fig. 8b) T cells (detected by FITC-labeled anti-Thyl.2) was analyzed.

The results obtained indicate that the *P. gingivalis*-treatment induced a harmful environment negatively affecting the newly administered normal T cells, as reflected by the downregulation of ζ expression observed in the donor T cells already 24 hours following injection. Moreover, the inventors proved that a harmful environment is generated in vivo and is mediated by the non-T cell population. *In vitro* mixing experiments were used, in which the non-T cell population isolated from the spleen of the *P. gingivalis*-treated mice was incubated with normal T cells. Within 24 hours of incubation, the normal T cells downregulated ζ expression, similar to what was observed in the in vivo experiments. These results indicate that the model system could be used for testing various immunotherapies as to their sensitivity to the harmful environment created in these mice. If the environment negatively affects the host's immune system, it should also affect the newly administered (donor's) T cells and shall prevent optimal response to vaccination protocols. Such immunotherapies would include T cell-mediated immunotherapies and vaccinations.

**Example 9**

**TLRs and their ligands in the induction of TCR ζ chain downregulation.**

Model system mice (C3H/Hen) were treated with LPS from *P.g.* (LPS-*P.g.*) or zymosan, as described above. Figure 10A shows FACS analysis histograms that represent the total expression of ζ-chain and CD3ε of gated splenic T cells (Thy1.2+ cells). Splenic T cells from zymosan-treated mice show a significant ζ chain down-regulation relative to the control mice, which were injected with adjuvant and OVA. Expression levels of CD3ε remain the same in both groups.

Further, in the results presented in Figure 10B, equal numbers of splenocytes from mice treated with TLR2 ligands, either LPS-P.g. or zymosan, and control mice were lysed and analyzed by immunoblotting with anti-Zap70, anti-CD3ε and anti-ζ antibodies. Splenic T cells from LPS-P.g. or zymosan treated mice (wt-TLR4 and mut-TLR4) show a significant ζ chain down-regulation relative to the control mice while the expression level of Zap70 and CD3ε remains the same. These results indicate that nonfunctional TLR4 (mut-TLR4) does not affect the function of TLR2.

Figure 10C shows Gr1+ and Mac1+ cells distribution in the spleen of zymosan-treated and control mice. Numbers in quadrants indicate the percentage of a specific cell population of the total splenocytes. Massive recruitment of Gr1+ and Mac1+ cells to the spleen of TLR$_2$ ligand (zymosan) treated mice [C3H/Hen (wt-tlr4) and C3H/Hej (mut tlr4)] while in the control mice this recruitment was not observed.

In Figure 10D, a lower percent of CFSE-labeled splenic T cells, that divided 1-4 times, following 48 hours activation with anti-CD3 and anti-CD28, in zymosan-treated mice (wt-TLR4 and mutTLR4) relative to splenic T cells from control mice (55%, 24%, 95%, respectively). While activation with PMA and ionophore, which activate signaling pathways downstream to the TCR, overcame partially this impaired function (78%, 81%, and 85%, respectively).

In Figure 10E, FACS analysis represents the percent of BrdU+ Thy1.2+ cells of total splenic T cells from control mice relative to splenic T cells from zymosan treated mice (wt-TLR4 and mut-TLR4) (67%, 14%, 9%, respectively). While activation with PMA and ionophore could partially overcome this impaired function (67%, 30%, 30%, respectively).

Figure 11A shows ζ chain downregulation in splenic T cells from C3H/Hen (wt-tlr4) mice treated with LPS from E.coli , in comparison with splenic T cells from control mice [C3H/Hen (wt-tlr4)] injected with adjuvant and OVA (gray) and TLR4 mutant mice [C3H/Hej(mutant tlr4)]. Equal numbers of splenocytes from LPS-coli treated mice and control mice were lysed and analyzed. Figure 11B shows immunoblot with anti-Zap70, anti-CD3ε and anti-ζ of splenic T cells from LPS-coli treated mice, control mice, and TLR-4 mutated mice [C3H/Hej(mutant tlr4)]. A significant downregulation can be seen in LPS-coli treated mice, which is not present in both controls. In Figure 11C, Grl+ and Macl+ cells distribution in the spleen of LPS-treated and non-treated mice, both from wild type (wt-tlr4) and mutant mice (mutant tlr4). Numbers in quadrants indicate the percentage of a specific cell population of the total splenocytes. Massive recruitment of Gr1+ and Mac1+ cells was observed to the spleen of TLR4 ligand-treated mice, which was not seen in the control mice.

Figure 11D shows how CFSE-labeled splenocytes were activated with anti-CD3ε and anti-CD28 (left column) or with combination of PMA and calcium ionophore (right column) for 60-72 hours. A lower percentage of CFSE-labeled splenic T cells from LPS-*E.coli* treated mice [C3H/Hen(wt-tlr4)], which divided 2-5 times following activation with anti-CD3 and anti-CD28, relative to splenic T cells from control mice or TLR4 mutated mice [C3H/Hej(mutant tlr4)] (21%, 96%, 94%, respectively). While activation with PMA and ionophore could overcome this impaired function (90%, 76%, 90%, respectively).

Figure 11E shows FACS analysis representing the percentage of BrdU+ Thy1.2+ cells in total splenic T cells from LPS-coli treated mice relative to splenic T cells from control mice or TLR4 mutated mice, following stimulation with anti-CD3 and anti-CD28 antibodies (15%, 76%, and 60%, respectively). While activation with PMA and ionophore could overcome this impaired function (36%, 62%, 66%).

In Figures 12A-E, treatment with TLR9 ligand induce specific ζ chain down-regulation and impaired T cell proliferation. Fig. 12A shows histograms of FACS analysis representing the total expression of ζ-chain and CD3ε of gated splenic T cells (Thy1.2+ cells) from TLR9 ligand (CpG-ODNs) treated mice (BALB/c) (black), compared to splenic T cells from control mice, which were injected with mutated CpG-ODNs (gray). Cells from TLR9 ligand (CpG-ODNs) treated mice show significant ζ chain down-regulation. Fig. 12B shows an immunoblot with anti-Zap70, anti-CD3ε and anti-ζ of splenic T cells from CpG-ODNs treated mice compared to control mice (injected with mutated CpG-ODN). These results also demonstrate the downregulation of ζ chain upon treatment with TLR ligands. Fig. 12C shows the result of CFSE-labeled splenocytes activated with anti-CD3ε and anti-CD28 (left column) or with combination of PMA and calcium ionophore (right column) for 60 hours. Following activation with anti-CD3 and anti-CD28, a lower percentage of CFSE-labeled splenic T cells, which divided 1-5 times, was found in CpG-treated mice relative to control mice (6% versus 84%, respectively). While activation with PMA and ionophore could overcome this impaired function (57%, 80%, respectively). Fig. 12D shows FACS data presenting BrdU incorporation in Thy-1.2+ spleen cells. A lower percentage of BrdU+ T cells of total T cells from CpG-treated mice relative to control mice following activation with anti-CD3 and anti-CD28 (9%, 71%, respectively) ζ or with ConA (39%, 83%, respectively). While activation with PMA and ionophore could overcome this impaired function (80%, 57%, respectively). Fig. 12E shows the results of three independent experiments, wherein T cells from CpG-treated mice show a significantly lower response to stimulation with anti-CD3 and Con A than do cells from control mice: *, $P < 0.002$ (Student's *t*-test).

It is important to emphasize that these results reproducibly indicate that ζ chain downregulation and impaired T cell function were not induced only following the *P. gingivalis* (heat-killed Gram negative bacteria), but also in the presence of other antigens. The results attest that the same phenomenon is observed upon treatment with heat-killed Gram positive bacteria, inactivated influenza virus, mycobacterium antigens and antigen (OVA) in complex with Toll-like receptor (TLR) ligands, such as CpG oligonucleotides (which activates via TLR9), purified E. coli LPS (which activates via TLR4), or purified *P. gingivalis* LPS or zymosan (which both activate via TLR4), and poly-IC that activate the TLR3 (data not shown). These results are summarized in Figures 10A-E, 11A-E and 12A-E, and indicate that, in addition to T cell activation due to antigen-dependent TCR triggering, there is also activation of other cells of the innate (macrophages, dendritic cells, NK cells) and adaptive (T cells, B cells) immune system, through various TLRs specifically expressed on various cells, depending on the molecular patterns (TLR ligands) and activated by the antigen in use. Based on these preliminary studies using antigens characteristic of various microorganisms, it is conceivable that TLR 2, 4, 9, 3 and potentially others that have not been yet studied, play a major role in the generation of a Th1-dependent inhibitory environment involved in the induction of ζ chain downregulation [Dabbagh, K. and Lewis, D. B. (2003) Curr Opin Infect Dis. 16:199-204; Johnson, G. B. et al. (2003) Trends Immunol. 24:19-24]. Moreover, some of the TLRs are also known to recognize mammalian endogenous molecules and their degradation products. These include heat-shock proteins released by stressed and/or necrotic cells and proteoglycans that are typically degraded during tissue diseases, e.g., tumor metastasis and necrotic events of the tissue [Cristofaro, P. and Opal, S. M. (2003) Expert. Opine Ther. Targets. 7:603-12]. A key role for the TLRs in the induction of ζ downregulation in various pathologies could therefore be predicted. Thus, as long as hosts (mice or human) are excessively exposed to antigens/adjuvants that induce chronic Th1-dependent inflammatory immune response involving TCR- and TLR-mediated activation processes, a pathogenic and immunosuppressive environment will ensue due to an unbalanced cytokine profile (reflected by the increased levels of IL12, IL23, IL27, IFNγ, TNFα) and secreted metabolites as those inducing oxidative stress (NO, $H_2O_2$). Such conditions could affect TCR ζ expression and T cell function.

## Example 10

**Involvement of Gr1+ Mac1+ myeloid cells in the generation of the immunosuppressive environment and in the induction of TCR ζ chain downregulation.**

Gr1+Mac1+ myeloid cells were separated from the spleen of *P. gingivalis*-treated and control mice and mixed for 24 hours with normal T cells or T cells isolated form *P. gangivalis*-treated.

The results presented in Figure 13A-D suggest that Gr1+Mac1+ myeloid cells as key players in the immunosuppressive environment generated in the mice suffering from chronic inflammation. These cells are involved in the induction of ζ chain downregulation (Figure 13A-D). Similar results were observed in cells from mice exposed to the various antigens and TLR ligands. In a developing inflammatory environment, the accumulation of regulatory CD11b+Gr-1+ myeloid suppressor cells (MSCs) is observed. This population is heterogeneous, and has been detected in lymphoid organs during tumor growth, in graft-versus-host reactions and in infectious diseases [Serafini, P. et al. (2004) Cancer Immunol. Immunother. 53, 64-72]. In all cases, impaired T cell responses to TCR-mediated stimuli are observed. MSCs play a key role in the regulation of the inflammatory process and in controlling T cell responses. One of the mechanisms used by MSCs to control T cells is based on arginine metabolism. MSCs use two enzymes to metabolize arginine: inducible nitric oxide synthase (NOS2), which generates nitric oxide (NO) and arginase 1 (ARG1), which converts arginine to urea and L-ornithine. NO inhibits T cell proliferation most likely by preventing activated T cells from entering the cell cycle [Mazzoni, A. et al. (2002) J. Immunol. 168, 689-95], and L-ornithine, which is consumed by the MSCs, is not available for the T cells that require it for cell proliferation and differentiation [Wu, G. & Morris, S.M., Jr. (1998) Biochem. J. 336, 1-17]. Thus, MSC recruitment can serve an important role in controlling excessive immune responses. The inventors demonstrate that expansion of MSCs in the spleen of infected or immunized mice is transient. By contrast, the number of MSCs under conditions of chronic inflammation such as in the present model system or during tumor progression and chronic infection remains high, and immunosuppression is maintained. MSCs suppress the activation of both CD8+ and CD4+ T cells, in an antigen and MHC-independent manner. Both cell-cell contact between MSCs and T cells, and secreted compounds are required to exert the inhibitory activity of MSCs [Jaffe, M.L. et al. (1996) Mol. Med. 2, 692-701 (1996)]. Thus, interference with the mechanisms used by MSCs to suppress T cell functions seems to be the most promising therapeutic strategy for restoring T cell function.

## Example 11

**Lymphocytes from lymph nodes of *P. gingivalis*-treated mice do not present ζ chain downregulation**

In a further experiment, the inventors set up to evaluate the ζ chain status in other lymphoid compartments, particularly the lymph nodes. Two test groups were established, the results of which are represented in Figures 14 and 15.

In the first test group, splenocytes from normal, untreated mice were isolated, stained with the red dye PKH and injected into the spleen of host mice, *P. gingivalis*-treated or control, untreated mice. Following injection, splenocytes (Fig. 14A) and lymph node cells (Fig. 14B) were harvested at days -2, -1, 1, 5 and 12 post-injection. The harvested cells were double stained with anti-Ty1.2 and anti-ζ antibodies, and analyzed by FACS. The analysis of ζ chain levels of the donor was performed by gating on Thy 1.2+ PKH+ cells. In parallel, the levels of ζ chain expression in the cells from spleen (Fig. 14C) and lymph nodes (Fig. 14D) from the donor were examined at the same time points. The results were obtained from a pool of 3-4 spleens, and 4-6 lymph nodes in each day (i.e., per time point). The results presented are of a representative experiment out of three.

In the second test group, splenocytes from *P. gingivalis*-treated mice were isolated, stained with PKH and injected into the spleen of host mice, *P. gingivalis*-treated or control, untreated mice. Following injection, splenocytes (Fig. 15A) and lymph node cells (Fig. 15B) were harvested at days -2, -1, 1, 5 and 12 post-injection. The harvested cells were double stained with anti-Ty1.2 and anti-ζ antibodies, and analyzed by FACS. The analysis of ζ chain levels of the donor was performed by gating on Thy 1.2+ PKH+ cells. In parallel, the levels of ζ chain expression in the cells from spleen (Fig. 15C) and lymph nodes (Fig. 15D) from the donor were examined at the same time points. The results were obtained from a pool of 3-4 spleens and 4-6 lymph nodes in each day (i.e., per time point). The results presented are of a representative experiment out of three.

The results show that ζ chain expression in T cells from the lymph nodes is not affected by exposure to the antigen (Fig. 14B, 15B and 15D). Moreover, spleen-derived T cells, which have been exposed to the antigen and then are exposed to the environment of the lymph node, wherein said lymph node is in an animal which has been exposed to the antigen or not, recover their ζ chain expression level (Fig. 15D). These results suggest that the lymph node is somehow an isolated environment which remains healthy, and which is not "spoiled" by the chronic inflammation condition induced in the whole animal. However, although the lymph node environment is normal, i.e. healthy, the T cells from peripheral blood and spleen, which show ζ chain downregulation and impaired function, are the ones who dictate the general impaired immune status of the host as indicated in Example 4.

**Claims**

1. Use of TCRζ chain protein level as a marker for an immunosuppressive environment in a subject suffering from a chronic inflammatory condition, wherein the detection of a downregulation of TCRζ chain protein level in a sample from said subject means the presence of an immunosuppressive environment in said subject.

2. Use of TCR ζ chain protein level as a prognostic marker for the emergence of an immunosuppressive environment in a subject suffering from a chronic inflammatory condition, wherein the detection of a downregulation of TCRζ chain protein level in a sample from said subject means the development of an immunosuppressive environment in said subject.

3. The use of claim 1 or 2 wherein said immunosuppressive environment is a result of any one of the following conditions: chronic inflammation, cancer, infections and autoimmune disorders.

4. The use of claim 1 or 2 wherein the sample is a blood or splenic T cell sample.

5. The use of claim 1 or 2 wherein only the TCRζ chain protein level is affected.

6. The use of claim 1 or 2 wherein the cells express normal amounts of surface $\alpha/\beta$-CD3.

7. The use of claim 1 or 2 wherein the expression of TCR- $\alpha$, TCR- $\beta$, CD3 $\gamma$, CD3 $\delta$ and CD3 $\epsilon$ is unchanged.

8. The use of claim 1 or 2 wherein said expression is detected by using labelled antibodies, optionally wherein the labelling is with biotin or a fluorescent dye, or wherein the detection is by FACS or Western blot analysis.

9. The use as defined in any one of claims 1-2, for monitoring of pre-immunotherapy patients.


**Patentansprüche**

1. Verwendung des TCRζ-Kettenproteinspiegels als Marker für eine immunsuppressive Umgebung in einem Individuum, das an einem chronischen Entzündungszustand leidet, wobei der Nachweis einer Abwärtsregulation des TCRζ-Kettenproteinspiegels in einer Probe aus dem Individuum die Anwesenheit einer immunsuppressiven Umgebung in dem Individuum bedeutet.

2. Verwendung des TCRζ-Kettenproteinspiegels als prognostischer Marker für das Auftreten einer immunsuppressiven Umgebung in einem Individuum, das an einem chronischen Entzündungszustand leidet, wobei der Nachweis einer Abwärtsregulation des TCRζ-Kettenproteinspiegels in einer Probe aus dem Individuum die Entwicklung einer immunsuppressiven Umgebung in dem Individuum bedeutet.

3. Verwendung nach Anspruch 1 oder 2, wobei die immunsuppressive Umgebung das Ergebnis von einer der folgenden Zustände ist: chronische Entzündung, Krebs, Infektionen und Autoimmunstörungen.

4. Verwendung nach Anspruch 1 oder 2, wobei die Probe eine T-Zellprobe aus Blut oder Milz ist.

5. Verwendung nach Anspruch 1 oder 2, wobei nur der TCRζ-Kettenproteinspiegel betroffen ist.

6. Verwendung nach Anspruch 1 oder 2, wobei die Zellen normale Mengen an Oberflächen-$\alpha/\beta$-CD3 exprimieren.

7. Verwendung nach Anspruch 1 oder 2, wobei die Expression von TCR-$\alpha$, TCR-$\beta$, CD3 $\gamma$, CD3 $\delta$ und CD3 $\epsilon$ unverändert ist.

8. Verwendung nach Anspruch 1 oder 2, wobei die Expression durch Verwendung von markierten Antikörpern nachgewiesen wird, wobei gegebenenfalls die Markierung mit Biotin oder einem Fluoreszenzfarbstoff erfolgt, oder wobei der Nachweis durch FACS- oder Western-Blot-Analyse erfolgt.

9. Verwendung nach einem der Ansprüche 1 bis 2 zum Überwachen von Patienten vor der Immuntherapie.

**Revendications**

1. Utilisation du niveau de la protéine chaîne TCR$\zeta$ en tant que marqueur pour un environnement immunosuppresseur chez un sujet souffrant d'un état inflammatoire chronique, pour laquelle la détection d'une régulation négative du niveau de la protéine chaîne TCR$\zeta$ dans un échantillon provenant dudit sujet signifie la présence d'un environnement immunosuppresseur dans ledit sujet.

2. Utilisation du niveau de la protéine chaîne TCR$\zeta$ en tant que marqueur pronostique de l'apparition d'un environnement immunosuppresseur chez un sujet souffrant d'un état inflammatoire chronique, pour laquelle la détection d'une régulation négative du niveau de la protéine chaîne TCR$\zeta$ dans un échantillon provenant dudit sujet signifie le développement d'un environnement immunosuppresseur chez ledit sujet.

3. Utilisation de la revendication 1 ou 2, pour laquelle ledit environnement immunosuppresseur est le résultat de l'un quelconque des états pathologiques suivants : inflammation chronique, cancer, infections et troubles auto-immuns.

4. Utilisation de la revendication 1 ou 2, pour laquelle l'échantillon est un échantillon de cellules T sanguines ou spléniques.

5. Utilisation de la revendication 1 ou 2, pour laquelle seul est affecté le niveau de la protéine chaîne TCR$\zeta$.

6. Utilisation de la revendication 1 ou 2, pour laquelle les cellules expriment des quantités normales d'$\alpha$/$\beta$-CD3 de surface.

7. Utilisation de la revendication 1 ou 2, pour laquelle l'expression du TCR-$\alpha$, du TCR-$\beta$, du CD3$\gamma$, du CD3$\delta$ et du CD3$\epsilon$ est inchangée.

8. Utilisation de la revendication 1 ou 2, pour laquelle ladite expression est détectée par utilisation d'anticorps marqués, en option pour laquelle le marquage s'effectue avec de la biotine ou un colorant fluorescent, ou pour laquelle la détection s'effectue par FACS ou par analyse par transfert de Western.

9. Utilisation telle que définie dans l'une quelconque des revendications 1-2, pour suivre des patients en pré-immunothérapie.

Fig. 1A

Fig. 1B

Fig. 1C

Fig. 1D

EP 1 708 565 B1

Fig. 1E

Fig. 1F

Fig. 2A

Fig. 2B

Fig. 3A

Fig. 3B

Fig. 3C

Fig. 3D

P. gingivalis/PBS
inj.

Chamb.
ins.

s.c    i.ch.    i.ch.

vir. inf.

D.: -21    -14    -7    0    +1    +4    +6    +9

Lu. col.

## Fig. 4A

## Fig. 4B

P. Gingivalls/PBS
inj.

Cham.
ins.

s.c.          i.ch.I          i.ch. II

-21          -14          -7          0

D.                          -8 | -6          | +1

Splen. col.

## Fig. 5A

| treat. | PBS | P. gingivalis | | | | |
|--------|-----|---------------|---|---|---|---|
| i.ch.II | + | − | − | − | − | + |
| i.ch.I | + | − | − | + | + | + |
| s.c. | + | + | + | + | + | + |
| d. harv. | +1 | -8 | +1 | -6 | +1 | +1 |

ζ →

ε →

## Fig. 5B

Fig. 6A

Fig. 6B

Fig. 6C

Fig. 7A

Fig. 7B

Fig. 7C

EP 1 708 565 B1

Fig. 8A

Fig. 8B

Fig. 8C

Fig. 9A

PKH-labeled donor cells
from normal mice

Donor cells within *P. gingivalis*-treated hosts

98.67%

1.33%

28.45%

71.55%

25.04%

74.96%

31.01%

68.99%

59.32%

40.68%

Intrasplenic injection
into *P. gingivalis*-treated
mice

TCR ζ exp.

49.17%

50.83%

84.72%

15.28%

34.36%

65.64%

34.36%

65.64%

88.12%

11.88%

**-2**

**-1**

**+1**

**+5**

**+12**

**D.**

Ho. *P.gingivalis*

Thy 1.2 exp.

Fig. 9B

Fig. 10A

Fig. 10B

Fig. 10C

Fig. 10D

Fig. 10E

## Fig. 11A

## Fig. 11B

Fig. 11C

Fig. 11D

Fig. 11E

Fig. 12A

Fig. 12B

Fig. 12C

Fig. 12D

Fig. 12E

Fig. 13A

Fig. 13B

Fig. 13C

Fig. 13D

Fig. 14A

Fig. 14B

Fig. 14C

Fig. 14D

Fig. 15A

Fig. 15B

Fig. 15C

Fig. 15D

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **WEISS, A. ; LITTMAN, D. R.** *Cell,* 1994, vol. 76, 263-74
- **KLAUSNER, R. D. et al.** *Annu Rev Cell Biol,* 1990, vol. 6, 403-31
- **MIZOGUCHI, H. et al.** *Science,* 1992, vol. 258, 1795-8
- **KANE, L. P. et al.** *Curr Opin Immunol,* 2000, vol. 12, 242-9
- **ALBEROLA-ILA, J. et al.** *Annu Rev Immunol,* 1997, vol. 15, 125-54
- **FINKE, J. H. et al.** *Cancer Res,* 1993, vol. 53, 5613-6
- **NAKAGOMI, H. et al.** *Cancer Res,* 1993, vol. 53, 5610-2
- **MATSUDA, M. et al.** *Int J Cancer,* 1995, vol. 61, 765-72
- **LAI, P. et al.** *Clin Cancer Res,* 1996, vol. 2, 161-73
- **KONO, K. et al.** *Clin Cancer Res,* 1996, vol. 2, 1825-8
- **KURT, R. A. et al.** *Int J Cancer,* 1998, vol. 78, 16-20
- **KUSS, I. et al.** *Clin Cancer Res,* 1999, vol. 5, 329-34
- **HEALY, C. G. et al.** *Cytometry,* vol. 32, 109-19
- **TRIMBLE, L. A. ; LIEBERMAN, J.** *Blood,* 1998, vol. 91, 585-94
- **ZEA, A. H. et al.** *Infect Immun,* 1998, vol. 66, 999-504
- **MAURICE, M. M. et al.** *J Immunol,* 1997, vol. 159, 2973-8
- **LIOSSIS, S. N. et al.** *J Clin Invest,* 1998, vol. 101, 1448-57
- **GENCO, C. A. ; ARKO, R. J.** *Methods Enzymol,* 1994, vol. 235, 120-40
- **HOURI-HADDAD, Y. et al.** *Immunology,* 2000, vol. 99, 215-20
- **COPE et al.** *Arthritis Res,* 2002, vol. 4 (3), 197-211
- **ISOMÄKI et al.** *J. Immunol.,* 2001, vol. 166, 5495-5507
- **HOURI-HADDAD, Y. et al.** *J Dent Res,* 2002, vol. 81, 395-8
- **CORREA, M. R. et al.** *J Immunol,* 1997, vol. 158, 5292-6
- **SUSSMAN, J. J. et al.** *Cell,* 1988, vol. 52, 85-95
- **ENYEDY, E. J. et al.** *Arthritis Rheum,* 2001, vol. 44, 1114-21
- **OTSUJI, M. et al.** *Proc Natl Acad Sci USA,* 1996, vol. 93, 13119-24
- **ALMAND, B. et al.** *J Immunol,* 2001, vol. 166, 678-89
- **KONO, K. et al.** *Eur J Immunol,* 1996, vol. 26, 1308-13
- **SCHMIELAU, J. et al.** *Cancer Res,* 2001, vol. 61, 4756-60
- **KUSMARTSEV, S. A. et al.** *Immunol,* 2000, vol. 165, 779-85
- **BAKER, P. J. et al.** *Arch Oral Biol,* 1994, vol. 39, 1035-40
- **KESAVALU, L. et al.** *Infect Immun,* 1992, vol. 60, 1455-64
- **HOURI-HADDAD et al.** *INFECT IMMUN,* 2000
- **FRANCO, J. L. et al.** *Cancer Res,* 1995, vol. 55, 3840-6
- **ROZDZIAL, M. M. et al.** *J Immunol,* 1994, vol. 153, 1563-80
- **SAMELSON, L.E. et al.** *J Immunol,* 1986, vol. 137, 3254-8
- **CAPLAN, S. ; BANIYASH, M.** *J Biol Chem,* 1996, vol. 271, 20705-12
- **HARLOW, E. ; LANE, D.** Antibodies: a laboratory manual. Cold Spring Harbor Laboratory Press, 1999
- **LESHEM, B. et al.** *J Immunol Methods,* 1999, vol. 223, 249-54
- **LESHEM, B. ; BRASS, D.** *Immunology,* 1998, vol. 95, 409-18
- **REED, J. L. ; MUENCH, M.** *Am. J. hyg.,* 1938, vol. 27, 493-497
- **FROLOV, I. et al.** *Immunology,* 1998, vol. 93, 923-8
- **BRONSTEIN-SITTON et al.** *J Biol Chem,* 1999, vol. 274, 23659-65
- **CAPLAN, S. et al.** *Proc Natl Acad Sci USA,* 1995, vol. 92, 4768-72
- **D'ORO, U. et al.** *Immunity,* 1997, vol. 7, 619-28
- **WELLS M. A. et al.** *J Immunol,* 1981, vol. 126, 1042-6
- **ADA, G. L. ; JONES, P. D.** *Top Microbiol Immunol,* 1986, vol. 128, 1-54
- **GERHARD, W. et al.** *Immunol Rev,* 1997, vol. 159, 95-103

- **SPELLBERG, B. ; EDWARDS, J. E., JR.** *Clin Infect Dis,* 2001, vol. 32, 76-102
- **DABBAGH, K. ; LEWIS, D. B.** *Curr Opin Infect Dis.,* 2003, vol. 16, 199-204
- **JOHNSON, G. B. et al.** *Trends Immunol.,* 2003, vol. 24, 19-24
- **CRISTOFARO, P. ; OPAL, S. M.** *Expert. Opine Ther. Targets,* 2003, vol. 7, 603-12
- **SERAFINI, P. et al.** *Cancer Immunol. Immunother,* 2004, vol. 53, 64-72
- **MAZZONI, A. et al.** *J. Immunol.,* 2002, vol. 168, 689-95
- **WU, G. ; MORRIS, S.M., JR.** *Biochem. J.,* 1998, vol. 336, 1-17
- **JAFFE, M.L. et al.** *Mol. Med.,* 1996, vol. 2, 692-701

SEQUENCE LISTING

<110> Yissum Research and Development Company of the Hebrew Universi ty of Jerusalem

<120> Model System for TCR zeta chain downregulation

<130> 16683/WO/03

<150> IL 158011
<151> 2003-09-18

<160> 2

<170> PatentIn version 3.1

<210> 1
<211> 22
<212> DNA
<213> Artificial Sequence

<220>
<223> CpG oligonucleotide

<400> 1

```
tgactgtgaa cgttcgagat ga
 22
```

<210> 2
<211> 22
<212> DNA
<213> Artificial Sequence

<220>

<223> mutated CpG oligonucleotide

<400> 2

```
tgactgtgaa ggttggagat ga
 22
```